(19) Europäisches Patentamt
European Patent Office
Office européen des brevets

(11) EP 2 070 937 A1

(12) EUROPEAN PATENT APPLICATION
published in accordance with Art. 153(4) EPC

(43) Date of publication:
17.06.2009 Bulletin 2009/25

(51) Int Cl.:
$C07H\ 5/02$ (2006.01)          $C07B\ 59/00$ (2006.01)
$G01T\ 1/161$ (2006.01)          $C07B\ 61/00$ (2006.01)

(21) Application number: 07806493.8

(22) Date of filing: 31.08.2007

(86) International application number:
PCT/JP2007/067020

(87) International publication number:
WO 2008/029734 (13.03.2008 Gazette 2008/11)

(84) Designated Contracting States:
AT BE BG CH CY CZ DE DK EE ES FI FR GB GR
HU IE IS IT LI LT LU LV MC MT NL PL PT RO SE
SI SK TR
Designated Extension States:
AL BA HR MK RS

(30) Priority: 06.09.2006 JP 2006241059

(71) Applicant: NIHON MEDI-PHYSICS CO., LTD.
Nishinomiya-shi, Hyogo 662-0918 (JP)

(72) Inventors:
• HIRANO, Keiichi
  Sodegaura-shi, Chiba 299-0266 (JP)
• ITO, Taku
  Sodegaura-shi, Chiba 299-0266 (JP)
• INO, Sento
  Tokyo 136-0075 (JP)

(74) Representative: Wibbelmann, Jobst
Wuesthoff & Wuesthoff
Patent- und Rechtsanwälte
Schweigerstrasse 2
81541 München (DE)

(54) PROCESS FOR PRODUCING RADIOACTIVE FLUORINE LABELED ORGANIC COMPOUND, AND RELEVANT SYNTHETIC APPARATUS AND PROGRAM

(57) A method of producing a radioactive-fluorine-labeled compound has a step of heating in a reaction vessel a mixture containing [$^{18}$F] fluoride ions, a phase transfer catalyst, potassium ions, and water to evaporate water from the mixture (S10), and in the above step has a step of measuring a temperature of an outlet tube for discharging evaporated water during the heating of the reaction vessel (S12), and the evaporation process is finished at a timing determined based on the result of temperature measurement in the step (S12) of measuring the temperature (S16). Consequently, the amount of water present in the mixture can be controlled to an appropriate range and a stable yield can be achieved in the method of producing a radioactive-fluorine-labeled organic compound.

【Fig. 3】

START

START EVAPORATION PROCESS — S10

PROCESS OF DETECTING PRESCRIBED CHANGE IN TEMPERATURE TREND (PARTICULAR TIME POINT) — S12

CALCULATE EVAPORATION FINISH TIME — S14

FINISH EVAPORATION PROCESS — S16

END

EP 2 070 937 A1

**Description**

RELATED APPLICATIONS

This application claims the benefit of Japanese Patent Application No. 2006-241059 filed on September 6, 2006 in Japan, the contents of which are incorporated herein by reference.

TECHNICAL FIELD

The present invention relates to a method of producing a radioactive-fluorine-labeled organic compound, a synthesizer for producing the compound, and a control program of the synthesizer.

BACKGROUND ART

Nuclear medicine examinations typified by positron emission tomography (hereinafter referred to as "PET") and single photon emission computed tomography (hereinafter referred to as "SPECT") are effective for diagnosis of cancer and other various diseases. These methods involve giving a medical agent labeled with a specific radioisotope (hereinafter referred to as a "radiopharmaceutical") and detecting gamma rays emitted directly or indirectly by the given medical agent. Nuclear medicine examinations have not only the great property of being highly specific and sensitive to diseases, but also a feature of being able to provide information on the function of lesions, which is not possessed by other examination methods.

For example, 2-[$^{18}$F]fluoro-2-deoxy-D-glucose (hereinafter referred to as "$^{18}$F-FDG") is one of radiopharmaceuticals to be used in PET examinations. Having a property of accumulating in regions where carbohydrate metabolism is high, $^{18}$F-FDG can specifically detect tumors in which carbohydrate metabolism is high.

PET provides high-quality images and can therefore provide images with higher diagnosis performance compared to SPECT, which has been widely used in clinical practice. For this reason, PET examinations are expected as a new diagnosis modality that follows SPECT examinations, and radiopharmaceuticals for PET examination use (hereinafter referred to as "PET diagnostic agents") have been developed by many research facilities and the like. For example, various receptor mapping agents and blood-flow diagnostic agents have been synthesized and researched for clinical application.

A PET diagnostic agent is a medical agent that contains as an active ingredient a compound labeled with a positron-emitting radionuclide $^{11}$C, $^{15}$O, $^{18}$F, or the like. The most widely used compound of these is an $^{18}$F-labeled organic compound typified by $^{18}$F-FDG. There have been proposed a variety of methods of producing $^{18}$F-FDG. Most of the methods of producing $^{18}$F-FDG are roughly classified into the method proposed by Hamacher (hereinafter referred to as the "Hamacher method") and on-column methods.

In the Hamacher method, a solution containing $^{18}$F, potassium carbonate, and a phase transfer catalyst is first evaporated to dryness to activate $^{18}$F. Then, the activated solution is added with an acetonitrile solution of a labeling precursor compound 1,3,4,6-tetra-O-acetyl-2-O-trifluoromethanesulfonyl-β-D-mannopyranose (hereinafter referred to as "TATM") and is heated to obtain an intermediate product 1,3,4,6-tetra-O-acetyl-2-[$^{18}$F]fluoro-2-deoxyglucose (hereinafter referred to as "$^{18}$F-TAFDG"). Subsequently, the $^{18}$F-TAFDG is subjected to deprotection and purification processes to obtain the target product $^{18}$F-FDG. In an on-column method, on the other hand, $^{18}$F-FDG is obtained by performing the activation of $^{18}$F and $^{18}$F labeling reaction in a column and performing the deprotection and purification. Methods of producing $^{18}$F-FDG are described, for example, in the following documents:

Japanese Patent Laid-Open Application No. Hei 6-157572;
Hamacher K., Coenen H. H., Stocklin G., "Efficient Stereospecific Synthesis of No-carrier-added-2-[18F]fluoro-2-deoxy-D-glucose Using Aminopolyether Supported Nucleophilic Substitution," J. Nucl. Med., 1986, 27, 2, pp. 235-238 (Document 1); and
K. Hamacher et al., "Computer-aided Synthesis (CAS) of No-carrier-added 2-[18F]Fluoro-2-deoxy-D-glucose: an Efficient Automated System for the Aminopolyether-supported Nucleophilic Fluorination," Applied Radiation and Isotopes (Great Britain), Pergamon Press, 1990, 41, 1, pp. 49-55 (Document 2).

It is disclosed that when $^{18}$F-FDG is synthesized by using the above methods, the solution containing $^{18}$F, potassium carbonate, and a phase transfer catalyst requires to be completely dehydrated in the process of evaporating the solution to dryness to activate $^{18}$F (see the above Document 1 and Published Japanese Translation of PCT International Application for Patent Application No. Hei 11-508923).

It is also disclosed that in the synthesis of an $^{18}$F-labeled organic compound, if removal of water in the evaporation process to activate $^{18}$F is insufficient, $^{18}$F may be hydrated to reduce the nucleophilicity of $^{18}$F, causing a reduction

in the yield of $^{18}$F-FDG (see Japanese Patent Laid-Open Application No. Hei 5-345731).

DISCLOSURE OF THE INVENTION

Problems to be solved by the invention

Among typical methods of producing $^{18}$F-FDG, the Hamacher method is characterized by being able to achieve a relatively high yield, but at the same time has a problem that the production yield may vary greatly. This variation is mainly caused by a variation in yield, i.e. the production yield of $^{18}$F-TAFDG, during the $^{18}$F labeling reaction (hereinafter referred to as a "radioactive fluorination yield"). Therefore, a stable commercial supply of $^{18}$F-FDG requires using a method that can achieve high-yield stable production, and this requires establishing conditions under which $^{18}$F-TAFDG can be produced stably at a high yield.

After studying methods of producing $^{18}$F-FDG, the inventors found that a stable and high radioactive fluorination yield can be achieved by making a certain amount of water be contained in the solution during the $^{18}$F labeling reaction, and suggested a preferred range of the amount of water in the solution (Japanese Patent Application No. 2005-352464).

However, a further device is required for controlling the amount of water in the solution to be within the above range in order to industrially produce $^{18}$F-FDG. That is, the amount of water in the solution can be determined experimentally by measuring using gas chromatographic analysis or the like, which on the other hand is a method that requires the evaporation process to be temporarily stopped to cool the solution to room temperature. Since the evaporation process cannot be interrupted in order to measure the amount of water in the solution when $^{18}$F-FDG is produced industrially, gas chromatographic analysis or the like cannot be used for industrial production of $^{18}$F-FDG.

A purpose of the invention made in view of the above-mentioned background is to provide a method of controlling the amount of water present in the mixture during the evaporation process to an appropriate range, with a simple configuration. Another purpose of the invention is to provide a production method, synthesizer control program, and synthesizer for a radioactive-fluorine-labeled organic compound that can provide a high radioactive fluorination yield by controlling to an appropriate range the amount of water present in the mixture containing $^{18}$F, potassium carbonate, and a phase transfer catalyst.

Means for solving the problems

As a result of keen experiments and examinations, the inventors have found that there is a correlation between the amount of water in the mixture and a temperature of an outer wall of an outlet tube for discharging evaporated water from a reaction vessel, and that the change in temperature of the outer wall of the outlet tube shows a certain trend in accordance with a decrease in the amount of water in the mixture. Based on these findings, the inventors have completed the invention in which in a process of heating in a reaction vessel a mixture containing [$^{18}$F] fluoride ions, a phase transfer catalyst, potassium ions, and water to evaporate water from the mixture, the amount of water remaining in the mixture is controlled to an appropriate range by finishing the evaporation process at a timing determined based on a temperature of the outer wall of the outlet tube. The inventors have also completed the invention in which a high radioactive fluorination yield can be achieved by applying the above method to a production method for $^{18}$F-FDG.

A method of producing a radioactive-fluorine-labeled compound of the invention comprises the steps of: heating in a reaction vessel a mixture containing [$^{18}$F] fluoride ions, a phase transfer catalyst, potassium ions, and water to evaporate water from the mixture; preparing a reaction solution including the mixture and a labeling precursor compound 1,3,4,6-tetra-O-acetyl-2-O-trifluoromethanesulfonyl-β-D-mannopyranose; and obtaining 1,3,4,6-tetra-O-acetyl-2-[$^{18}$F] fluoro-2-deoxyglucose by giving reaction conditions to the reaction solution, where the step of evaporating water has a step of measuring a temperature of an outlet tube for discharging evaporated water from the reaction vessel to determine a finish timing of the evaporation based on the measured temperature, and the evaporation is finished at the finish timing.

In the above method of producing a radioactive-fluorine-labeled compound, in the step of determining a finish timing of the evaporation, a temperature of an outer wall of the outlet tube may be measured.

In the above method of producing a radioactive-fluorine-labeled compound, in the step of determining a finish timing of the evaporation, a finish timing of the evaporation may be determined based on a change point at which a trend in the measured temperature, after changed from up to down, changes again to up.

In the above method of producing a radioactive-fluorine-labeled compound, in the step of determining a finish timing of the evaporation, a finish timing of the evaporation may be determined based on a point at which, during a period from a time when a trend in the measured temperature changes from up to down to a time when the trend changes again to up, the negative gradient of the change in temperature is maximum.

A method of producing a radioactive-fluorine-labeled compound of another aspect of the invention comprises the steps of: heating in a reaction vessel a mixture containing [$^{18}$F] fluoride ions, a phase transfer catalyst, potassium ions, and water to evaporate water from the mixture; preparing a reaction solution including the mixture and a labeling precursor compound 1,3,4,6-tetra-O-acetyl-2-O-trifluoromethanesulfonyl-β-D-mannopyranose; and obtaining 1,3,4,6-tetra-O-acetyl-2-[18F]fluoro-2-deoxyglucose by giving reaction conditions to the reaction solution, where, in the step of evaporating water, the evaporation is finished at a predetermined evaporation finish timing, and where, as the evaporation finish timing, a timing is used that is determined by measuring a temperature of an outlet tube for discharging evaporated water from the reaction vessel and being based on the measured temperature.

A method of producing a radioactive-fluorine-labeled compound of another aspect of the invention comprises the steps of: heating in a reaction vessel a mixture containing [18F] fluoride ions, a phase transfer catalyst, potassium ions, and water to evaporate water from the mixture; preparing a reaction solution including the mixture and a labeling precursor compound 1,3,4,6-tetra-O-acetyl-2-O-trifluoromethanesulfonyl-β-D-mannopyranose; and obtaining 1,3,4,6-tetra-O-acetyl-2-[18F]fluoro-2-deoxyglucose by giving reaction conditions to the reaction solution, where the step of evaporating water has a step of measuring a temperature of a component connected to the reaction vessel to determine a finish timing of the evaporation based on the measured temperature, and the evaporation is finished at the finish timing.

A method of controlling water content of a mixture containing [18F] fluoride ions, a phase transfer catalyst, potassium ions, and water of the invention comprises the steps of: heating in a reaction vessel a mixture containing [18F] fluoride ions, a phase transfer catalyst, potassium ions, and water to start evaporation of water from the mixture; measuring a temperature of an outlet tube for discharging evaporated water from the reaction vessel to determine a finish timing of the evaporation based on the measured temperature; and finishing the evaporation of water at the finish timing.

In the above method of controlling water content of the mixture, in the step of determining a finish timing of the evaporation, a temperature of an outer wall of the outlet tube may be measured.

In the above method of controlling water content of the mixture, in the step of determining a finish timing of the evaporation, a finish timing of the evaporation may be determined based on a change point at which a trend in the measured temperature, after changed from up to down, changes again to up.

In the above method of controlling water content of the mixture, in the step of determining a finish timing of the evaporation, a finish timing of the evaporation may be determined based on a point at which, during a period from a time when a trend in the measured temperature changes from up to down to a time when the trend changes again to up, the negative gradient of the change in temperature is maximum.

A method of controlling water content of a mixture containing [18F] fluoride ions, a phase transfer catalyst, potassium ions, and water of another aspect of the invention comprises the steps of: heating in a reaction vessel a mixture containing [18F] fluoride ions, a phase transfer catalyst, potassium ions, and water to start evaporation of water from the mixture; and finishing the evaporation of water at a predetermined evaporation finish timing, where, as the evaporation finish timing, a timing is used that is determined by measuring a temperature of an outlet tube for discharging evaporated water from the reaction vessel and being based on the measured temperature.

A method of controlling water content of a mixture containing [18F] fluoride ions, a phase transfer catalyst, potassium ions, and water of another aspect of the invention comprises the steps of: heating in a reaction vessel a mixture containing [18F] fluoride ions, a phase transfer catalyst, potassium ions, and water to start evaporation of water from the mixture; measuring a temperature of a component connected to the reaction vessel to determine a finish timing of the evaporation based on the measured temperature; and finishing the evaporation of water at the finish timing.

A synthesizer of the invention comprises: a reaction vessel; a heater for heating the reaction vessel in order to evaporate water from a mixture contained in the reaction vessel; an outlet tube for discharging evaporated water from the reaction vessel; and a thermometer for measuring a temperature of the outlet tube.

In the above synthesizer, the thermometer may measure a temperature of an outer wall of the outlet tube. The thermometer may measure a temperature of an inner wall or inside of the outlet tube.

In the above synthesizer, the thermometer may be attached to the outlet tube at a position within 30 cm of a connection between the outlet tube and the reaction vessel.

The above synthesizer may have a controller for controlling the heater based on a temperature measured by the thermometer.

In the above synthesizer, the controller may control the heater such that water content of the mixture is within a prescribed range of values, based on a trend in temperature measured by the thermometer.

In the above synthesizer, for synthesizing 18F-FDG, the controller may control the heater in such a way as to determine a finish timing to finish the evaporation based on a change point at which a trend in temperature measured by the thermometer, after changed from up to down, changes again to up, and as to finish the evaporation at the finish timing.

In the above synthesizer, for synthesizing 18F-FDG, the controller may control the heater in such a way as to determine a finish timing to finish the evaporation based on a point at which, during a period from a time when a trend in temperature measured by the thermometer changes from up to down to a time when the trend changes again to up, the negative gradient of the change in temperature is maximum, and as to finish the evaporation at the finish timing.

A synthesizer according to another aspect of the invention comprises: a reaction vessel; a heater for heating the reaction vessel in order to evaporate water from a mixture contained in the reaction vessel; a thermometer for measuring a temperature of a component connected to the reaction vessel; and a controller for controlling the heater based on a temperature measured by the thermometer.

A program of the invention is for evaporating water from a solution containing [$^{18}$F] fluoride ions, a phase transfer catalyst, and potassium ions by means of a synthesizer comprising: a reaction vessel; a heater for heating the reaction vessel in order to evaporate water from a solution contained in the reaction vessel; an outlet tube for discharging water evaporated from within the reaction vessel; and a thermometer for measuring a temperature of the outlet tube, and the program causes the synthesizer to execute the steps of: heating the reaction vessel by means of the heater to start the evaporation; acquiring information on a temperature of the outlet tube from the thermometer; determining a finish timing of the evaporation based on the temperature; and finishing the evaporation at the finish timing.

In the above program, in the step of determining a finish timing of the evaporation, a finish timing of the evaporation may be determined based on a change point at which a trend in the acquired temperature, after changed from up to down, changes again to up.

In the above program, in the step of determining a finish timing of the evaporation, a finish timing of the evaporation may be determined based on a point at which, during a period from a time when a trend in the acquired temperature changes from up to down to a time when the trend changes again to up, the negative gradient of the change in temperature is maximum.

A program of another aspect of the invention is for evaporating water from a solution containing [$^{18}$F] fluoride ions, a phase transfer catalyst, and potassium ions by means of a synthesizer comprising: a reaction vessel; a heater for heating the reaction vessel in order to evaporate water from a solution contained in the reaction vessel; and a thermometer for measuring a temperature of a component connected to the reaction vessel, and the program causes the synthesizer to execute the steps of: heating the reaction vessel by means of the heater to start the evaporation; acquiring information on a temperature of the component from the thermometer; determining a finish timing of the evaporation based on the temperature; and finishing the evaporation at the finish timing.

In the invention, in the process of evaporating water from the mixture, the amount of water remaining in the mixture can be controlled to an appropriate range. By applying this evaporation method to the production of a radioactive-fluorine-labeled organic compound, the radioactive fluorination yield can be stably increased.

There are other aspects of the invention as described below. This disclosure of the invention therefore intends to provide part of the invention and does not intend to limit the scope of the invention described and claimed herein.

BRIEF DESCRIPTION OF THE DRAWINGS

Figure 1 shows a configuration of a synthesizer of an embodiment of the invention;
Figure 2 shows a configuration of a controller;
Figure 3 is a flowchart showing an example of a processing flow in a method of controlling water content of the embodiment;
Figure 4 shows a change in temperature of an outlet tube during an evaporation process;
Figure 5 is a flowchart for detecting a point at which a negative gradient is maximum;
Figure 6 is a flowchart showing a process of detecting a local maximum value;
Figure 7 is a flowchart showing a process of detecting a maximum negative gradient;
Figure 8 is a flowchart for detecting a point at which a gradient becomes a certain proportion of a maximum gradient or less;
Figure 9 is a flowchart showing in detail the process of detecting a point at which a gradient becomes a certain proportion of a maximum gradient or less;
Figure 10 shows the relation between time elapsed from a maximum negative gradient and water content of a sample; and
Figure 11 shows the relation between time elapsed from a time point at which a gradient indicates 1/10 of a maximum negative gradient and water content of a sample.

BEST MODE OF EMBODYING THE INVENTION

Now, a synthesizer and a method of producing a radioactive-fluorine-labeled compound according to an embodiment of the invention will be described with reference to the drawings. It will be understood that the embodiments described below are only examples of the invention, and the invention can be varied in various aspects. Therefore, the specific configurations and functions disclosed below do not limit the claims.

[Configuration of the synthesizer]

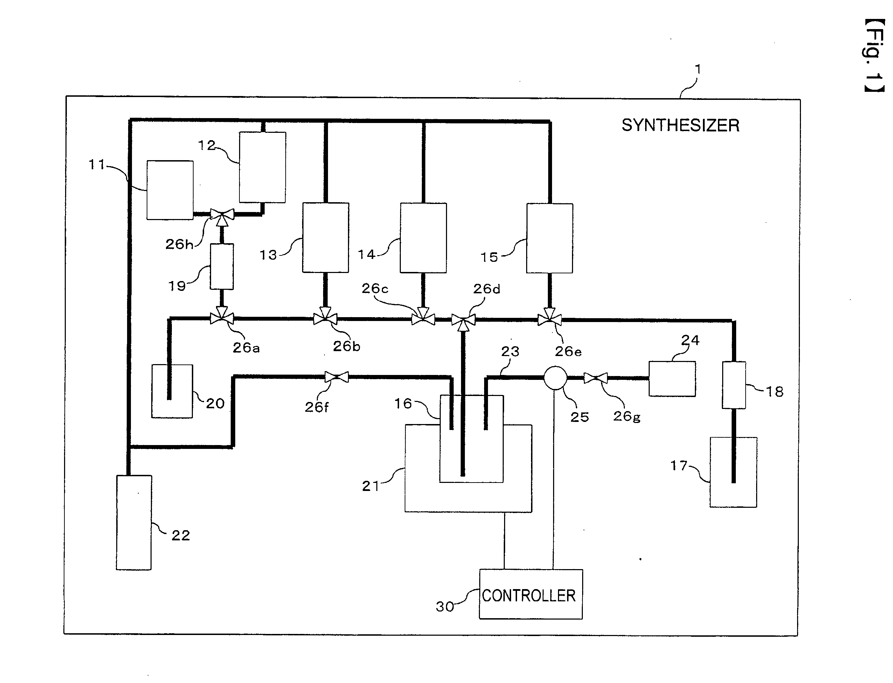

Figure 1 shows a configuration of a synthesizer 1 of an embodiment of the invention. The synthesizer 1 comprises reagent vessels 11 to 15 for storing required reagents and materials, a reaction vessel 16 for synthesizing from the reagents, and an $^{18}$F-FDG recovery vessel 17 for recovering $^{18}$F-FDG generated in the reaction vessel 16. The reagent vessels 11 to 15, the reaction vessel 16, and the $^{18}$F-FDG recovery vessel 17 are connected to one another by piping. A purification column 18 is provided on the piping between the reaction vessel 16 and the $^{18}$F-FDG recovery vessel 17.

In the synthesizer 1, the reagent vessel 11 is packed with $H_2$$^{18}$O-enriched water including [$^{18}$F] fluoride ions; the reagent vessel 12 is packed with a potassium carbonate solution; the reagent vessel 13 is packed with a phase transfer catalyst; the reagent vessel 14 is packed with an acetonitrile solution of TATM; and the reagent vessel 15 is packed with hydrochloric acid, as reagents for producing $^{18}$F-FDG.

The reagent vessel 11 is connected with an anion exchange column 19. The anion exchange column 19 is connected to the reaction vessel 16 and an $^{18}$O-enriched water recovery vessel 20 by piping. The $H_2$$^{18}$O-enriched water including [$^{18}$F] fluoride ions packed in the reagent vessel 11 is passed through the anion exchange column 19. The $^{18}$O-enriched water passed through the anion exchange column 19 is recovered by the $^{18}$O-enriched water recovery vessel 20. The passing of $^{18}$O-enriched water allows the anion exchange column 19 to adsorb and collect $^{18}$F, which is introduced into the reaction vessel 16 by passing potassium carbonate in a next process.

The synthesizer 1 has a heater 21 for heating the reaction vessel 16. The synthesizer 1 also has a helium cylinder 22 for feeding helium gas into the reagent vessels 11 to 15, the reaction vessel 16, and the like.

The reaction vessel 16 is provided with an outlet tube 23 for discharging water evaporated in a process of evaporating a reaction solution. The outlet tube 23 is provided with a vacuum pump 24, which sucks water evaporated from a reaction solution to the outside of the reaction vessel 16.

The synthesizer 1 of the embodiment has a thermometer 25 on the outlet tube 23 for measuring a temperature of an outer wall of the outlet tube 23. The thermometer 25 is positioned within 30 cm of a connection between the reaction vessel 16 and the outlet tube 23, preferably positioned within 10 cm of the connection, more preferably positioned within 5 cm of the connection, and particularly preferably positioned 0.5 to 1.5 cm from the connection. Providing the thermometer 25 near the reaction vessel 16 in this way allows to measure the temperature of a position that is susceptible to water evaporated from within the reaction vessel 16. Consequently, a point at which a trend in temperature changes can be detected accurately.

The synthesizer 1 has a plurality of valves 26a to 26h on the piping connecting the reagent vessels 11 to 15, the reaction vessel 16, the $^{18}$F-FDG recovery vessel 17, and the like. Used as the valves 26a to 26h are three-way valves 26a to 26e and 26h that are placed on branches of the piping, and open/close valves 26f and 26g that are placed on unbranched parts of the piping.

The synthesizer 1 has a controller 30 for controlling the above various components in the synthesizer 1. The controller 30 has functions to, for example, instruct the heater 21 to start and finish heating, and control open/close of each valve 26a to 26h.

[Configuration of the controller]

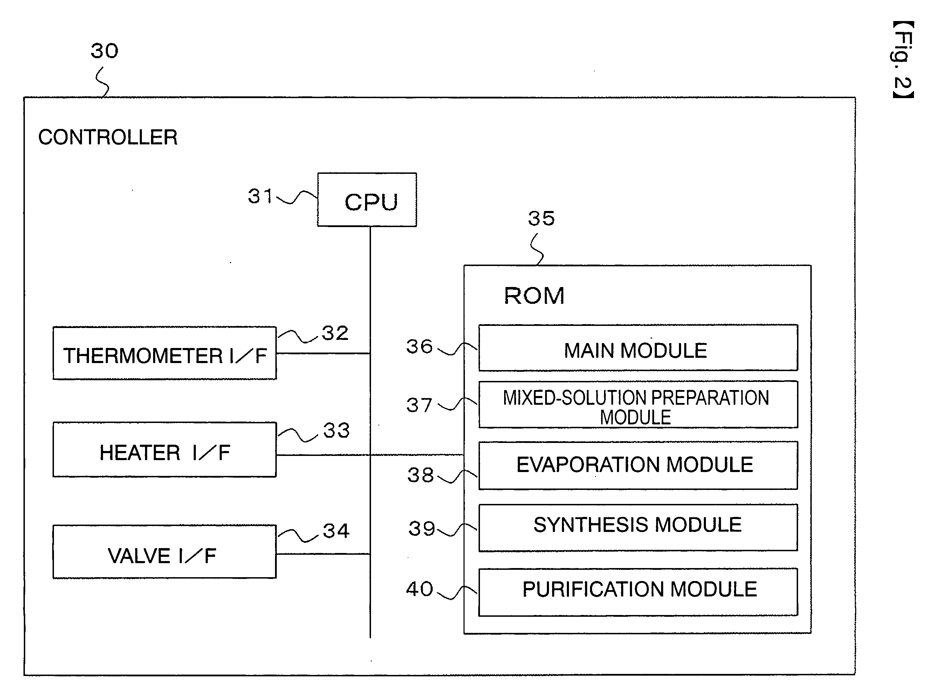

Figure 2 shows a configuration of the controller 30. The controller 30 has a thermometer interface 32 for acquiring information on temperature from the thermometer 25, a heater interface 33 for transmitting instructions to start and finish heating to the heater 21, and a valve interface 34 for transmitting instructions to the valves 26a to 26h. The controller 30 is connected with the thermometer 25 via the thermometer interface 32, and connected with the heater 21 via the heater interface 33. The controller 30 is connected with each valve 26a to 26h via the valve interface 34, though this is not shown in Figure 1. The controller 30 has a function to control the synthesizer 1 and controls not just the heater 21, the thermometer 25, and the valves 26a to 26h, but also various components in the synthesizer 1, in the same way as conventional synthesizers.

The controller 30 has a ROM 35 storing a program for controlling a process of producing a radioactive-fluorine-labeled compound. The ROM 35 stores a main module 36, a mixed-solution preparation module 37, an evaporation module 38, a synthesis module 39, and a purification module 40. While an example in which each module 36 to 40 is stored in the ROM 35 is described here, the program need not be stored in the ROM 35 but also may be stored in a hard disk. The program may be recorded on an external recording medium, such as a CD-ROM and a floppy disk (registered trademark). In this case, inserting a recording medium on which the program is recorded into a reader (not shown) provided on the controller 30 allows a CPU 31 to access the program. Control based on the program recorded on the recording medium allows the synthesizer 1 to produce a radioactive-fluorine-labeled compound.

The ROM 35 and each interface 32 to 34 are connected to the CPU 31 via a bus. The CPU 31 reads the modules stored in the ROM 35, performs a calculation process according to the read program, and thereby controls the synthesizer 1 to produce a radioactive-fluorine-labeled compound.

Each module 36 to 40 stored in the ROM 35 of the controller will next be described. The main module 36 is a module for controlling the order of execution of the mixed-solution preparation module 37, evaporation module 38, synthesis module 39, and purification module 40.

The mixed-solution preparation module 37 has a function to control the valves 26a to 26h and the like of the synthesizer 1 to prepare a mixed solution including a phase transfer catalyst, [$^{18}$F] fluoride ions, and potassium ions.

The process of preparing the mixed solution performed by the synthesizer 1 will be described with reference to Figure 1. First, the synthesizer 1 passes $H_2$$^{18}$O-enriched water including [$^{18}$F] fluoride ions from the reagent vessel 11 via the three-way valve 26h through the anion exchange column 19, and recovers the water via the three-way valve 26a in the $^{18}$O-enriched water recovery vessel 20. This allows the [$^{18}$F] fluoride ions to be adsorbed and collected by the anion exchange column 19 and be separated from the $H_2$$^{18}$O-enriched water collected in the $^{18}$O-enriched water recovery vessel 20. After that, the synthesizer 1 opens the valve 26g, and opens the three-way valves 26a, 26b, 26c, and 26d to open the passage between the exit side of the anion exchange column 19 and the reaction vessel 16. Under this condition, the synthesizer 1 pours the potassium carbonate solution from the reagent vessel 12 into the anion exchange column 19 to elute the [$^{18}$F] fluoride ions into the reaction vessel 16. Then, the synthesizer 1 closes the three-way valve 26a to close the passage between the reaction vessel 16 and the reagent vessels 11 and 12, and opens the three-way valve 26b to open the passage between the reagent vessel 13 and the reaction vessel 16, adding the phase transfer catalyst from the reagent vessel 13 to the reaction vessel 16. The mixed-solution preparation module 37 causes the synthesizer 1 to execute these series of processes.

The evaporation module 38 has a function to control the valves 26a to 26h, heater 21, and the like of the synthesizer 1 to evaporate water from a mixture including a phase transfer catalyst, [$^{18}$F] fluoride ions, potassium ions, and water. The process of evaporation performed by the synthesizer 1 will be described with reference to Figure 1. The synthesizer 1 closes the three-way valve 26d to close the passage between the reaction vessel 16 and the reagent vessels 13 to 15 and the like, opens the valve 26g, and starts to heat the reaction vessel 16 using the heater 21. After the heating of the reaction vessel 16 is started, the controller 30 acquires information on a temperature measured by the thermometer 25, and determines a timing to finish the evaporation process based on the acquired temperature information. The synthesizer 1 then stops the heating by the heater 21 at the determined evaporation finish timing, and closes the valve 26g to stop the evaporation process. The evaporation module 38 causes the synthesizer 1 to execute these series of processes.

In addition to the above-described heating operation, the heating process may involve an operation in which helium gas is introduced from the helium cylinder 22 via the valve 26f into the reaction vessel 16. In this case, the synthesizer 1 closes the three-way valve 26d to close the passage between the reaction vessel 16 and the reagent vessels 13 to 15 and the like, opens the valve 26g while opening the valve 26f to feed helium gas to the reaction vessel 16, and starts to heat the reaction vessel 16 using the heater 21. After that, the synthesizer 1 stops the heating by the heater 21 at an evaporation finish timing determined by the same method as above, closes the valve 26f to stop the feed of helium gas to the reaction vessel 16, and further closes the valve 26g to stop the evaporation process.

The synthesis module 39 has a function to control the valves 26a to 26h, heater 21, and the like of the synthesizer 1 to synthesize the target, $^{18}$F-FDG. The synthesis process performed by the synthesizer 1 will be described with reference to Figure 1. First, the synthesizer 1 opens the three-way valves 26c and 26d to open the passage between the reagent vessel 14 and the reaction vessel 16. The synthesizer 1 then applies pressure to the reagent vessel 14 using the helium cylinder 22, thereby introducing the TATM solution of the reagent vessel 14 into the reaction vessel 16. When the introduction of the TATM solution into the reaction vessel 16 is finished, the synthesizer 1 finishes the pressurization with helium gas, and closes the three-way valve 26d to close the passage from the reaction vessel 16 to the reagent vessels 11 to 15. The synthesizer 1 then closes the valve 26g, uses the heater 21 to heat the above reaction solution to give reaction conditions thereto, and synthesizes $^{18}$F-TAFDG by nucleophilic substitution reaction. Subsequently, the synthesizer 1 opens the valve 26g, and heats the reaction vessel 16 further under that condition to substantially evaporate the solvent from the reaction solution. The synthesizer 1 then opens the three-way valves 26d and 26e and applies pressure to the reagent vessel 15 using the helium cylinder 22 under that condition to introduce the hydrochloric acid of the reagent vessel 15 into the reaction vessel 16. The synthesizer 1 closes the valves 26d and 26g to seal the reaction vessel 16, and heats the reaction vessel 16 using the heater 21, thereby carrying out acid hydrolysis. The synthesis module 39 causes the synthesizer 1 to execute these series of processes.

In addition to the above-described heating operation, the above process of evaporating the solvent may involve an operation in which helium gas is introduced from the helium cylinder 22 via the valve 26f into the reaction vessel 16. In this case, after finishing the synthesis of $^{18}$F-TAFDG, the synthesizer 1 opens the valve 26g while opening the valve 26f to feed helium gas to the reaction vessel 16, and substantially evaporates the solvent from the reaction solution in the same manner as above. Then, after introducing the hydrochloric acid of the reagent vessel 15 into the reaction vessel 16 in the same operation as above, the synthesizer 1 closes the valves 26d, 26f, and 26g to seal the reaction vessel 16, and carries out acid hydrolysis in the same manner as above.

As the reaction conditions and amount of reagents in the above processes can be used known conditions (e.g.

methods described in documents (Radioisotopes, 50 (2001), pp. 205-227; Radioisotopes, 50 (2001), pp. 228-256; and Production and quality control of radioactive agents for PET - A guideline to synthesis and clinical use (PET you houshasei yakuzai no seizou oyobi hinshitsu kanri - Gousei to rinshou shiyou heno tebiki), 2nd Edition, edited by PET Kagaku Workshop)).

The purification module 40 has a function to control the valves 26a to 26h and the like of the synthesizer 1 to cause the synthesized $^{18}$F-FDG to be purified. The purification process performed by the synthesizer 1 will be described with reference to Figure 1. First, the synthesizer 1 opens the three-way valves 26d and 26e to open the passage between the reaction vessel 16 and the purification column 18. The synthesizer 1 also opens the valve 26f and uses the helium cylinder 22 to apply pressure to the reaction vessel 16, thereby passing the reaction solution from the reaction vessel 16 through the purification column 18 to recover it in the $^{18}$F-FDG recovery vessel 17. The purification module 40 causes the synthesizer 1 to execute these series of processes.

As the conditions and purification column 18 to be used in the above processes can be used known conditions (e.g. methods described in documents (Radioisotopes, 50 (2001), pp. 205-227; Radioisotopes, 50 (2001), pp. 228-256; and Production and quality control of radioactive agents for PET - A guideline to synthesis and clinical use (PET you houshasei yakuzai no seizou oyobi hinshitsu kanri - Gousei to rinshou shiyou heno tebiki), 2nd Edition, edited by PET Kagaku Workshop)).

[Production method for $^{18}$F-FDG]

A method of producing $^{18}$F-FDG by means of the synthesizer 1 of the embodiment of the invention will be described next.

First, reagents are introduced into the reagent vessels 11 to 15 of the synthesizer 1. Specifically, the reagent vessel 11 is packed with $H_2{}^{18}O$-enriched water including [$^{18}$F] fluoride ions; the reagent vessel 12 is packed with a potassium carbonate solution; the reagent vessel 13 is packed with a phase transfer catalyst; the reagent vessel 14 is packed with an acetonitrile solution of TATM; and the reagent vessel 15 is packed with hydrochloric acid.

Then, the controller 30 reads and executes the mixed-solution preparation module 37, thereby causing the synthesizer 1 to prepare a mixture including the phase transfer catalyst, [$^{18}$F] fluoride ions, potassium ions, and water.

The synthesizer 1 passes the $H_2{}^{18}O$-enriched water including [$^{18}$F] fluoride ions from the reagent vessel 11 via the three-way valve 26h through the anion exchange column 19, and recovers the water via the three-way valve 26a in the $^{18}$O-enriched water recovery vessel 20. This process causes the [$^{18}$F] fluoride ions to be adsorbed and collected by the anion exchange column 19 and be separated from the $H_2{}^{18}O$-enriched water collected in the $^{18}$O-enriched water recovery vessel 20. Then, the synthesizer 1 opens the valve 26g, and opens the three-way valves 26a, 26b, 26c, and 26d to open the passage between the exit side of the anion exchange column 19 and the reaction vessel 16. Under this condition, the synthesizer 1 pours the potassium carbonate solution from the reagent vessel 12 into the anion exchange column 19 to elute the [$^{18}$F] fluoride ions into the reaction vessel 16. Then, the synthesizer 1 closes the three-way valve 26a to close the passage between the anion exchange column 19 and each reagent vessel 13 to 15, the reaction vessel 16, and the like. The synthesizer 1 then opens the three-way valve 26b to open the passage between the reagent vessel 13 and the reaction vessel 16, adding the phase transfer catalyst from the reagent vessel 13 to the reaction vessel 16.

In the above, the mixture including the phase transfer catalyst, [$^{18}$F] fluoride ions, potassium ions, and water can be obtained according to usual methods (e.g. methods described in documents (Radioisotopes, 50 (2001), pp. 205-227; Radioisotopes, 50 (2001), pp. 228-256; and Production and quality control of radioactive agents for PET - A guideline to synthesis and clinical use (PET you houshasei yakuzai no seizou oyobi hinshitsu kanri - Gousei to rinshou shiyou heno tebiki), 2nd Edition, edited by PET Kagaku Workshop)).

The amounts of potassium carbonate and phase transfer catalyst to be used here can be the amounts used usually in producing $^{18}$F-FDG (or $^{18}$F-TAFDG). As the phase transfer catalyst can be used a catalyst used usually in producing $^{18}$F-FDG (or $^{18}$F-TAFDG).

After the mixture including the phase transfer catalyst, [$^{18}$F] fluoride ions, potassium ions, and water is prepared in the reaction vessel 16, the controller 30 reads and executes the evaporation module 38, thereby applying an evaporation process to the mixture to control water content of the mixture.

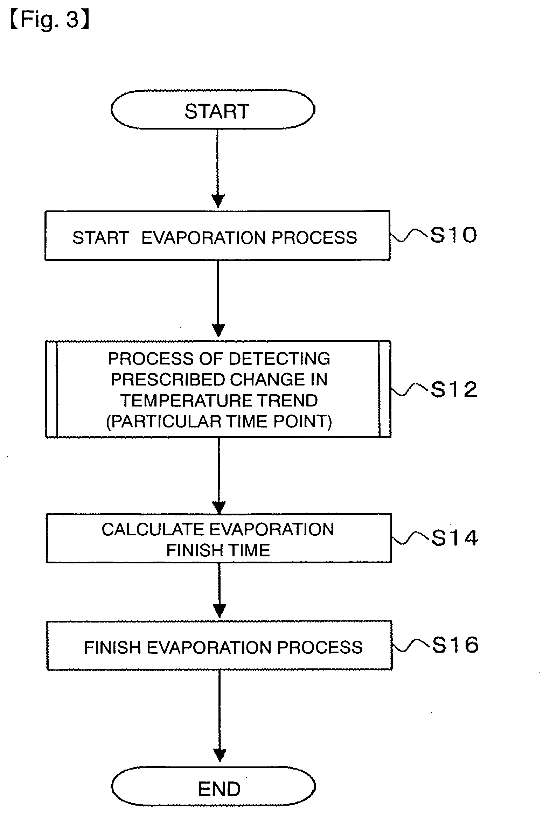

Figure 3 shows an operation of the evaporation process of the synthesizer 1. First, the mixture including the phase transfer catalyst, [$^{18}$F] fluoride ions, potassium ions, and water is heated in the reaction vessel 16 to start evaporation (S10). Describing with reference to Figure 1, the synthesizer 1 closes the three-way valve 26d to close the passage between the reaction vessel 16 and the reagent vessels 11 to 15, and at the same time opens the valve 26g. Under that condition, the synthesizer 1 starts to heat the reaction vessel 16 using the heater 21 (S10).

During the heating and evaporation process started in the above-described step S10, the controller 30 observes a trend in temperature acquired from the thermometer 25 and performs a process of detecting a particular time at which the trend indicates a prescribed change (hereinafter referred to as a "particular time point") (S12).

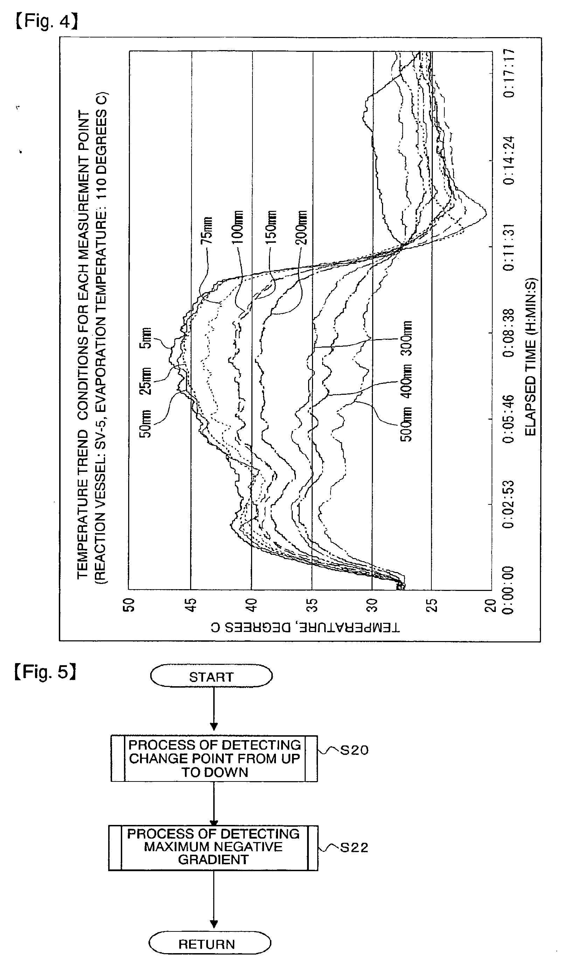

Figure 4 shows a change in temperature of the outlet tube 23 during the evaporation process. In the synthesizer 1 of the embodiment, a temperature of the outlet tube 23 has a trend in which it rises from the start of the evaporation process, then falls, and then rises again. In the change in temperature having such a trend, the particular time point to be detected by the controller 30 is, for example: (1) a change point at which a trend in temperature, after changed from up to down, changes again to up; or (2) a point at which, during a period from a time when a trend in temperature changes from up to down to a time when the trend changes again to up, the negative gradient is maximum. Various methods are conceivable as the method of the controller 30 detecting a particular time point based on an acquired temperature, and any one of the methods can be adopted. Several methods will be described later.

After detecting a particular time point, the controller 30 determines a finish timing of the evaporation process based on the particular time point (S14). Concrete methods for determining a finish timing of the evaporation based on a particular time point differ depending on the heating temperature of the heater 21, the material of the outlet tube 23, the position where the thermometer 25 is located, and the like.

The synthesizer 1 stops the heating by the heater 21 at the determined finish timing of the evaporation, and closes the valve 26g to finish the evaporation process (S16). In this way, water content of the mixture containing the [$^{18}$F] fluoride ions, phase transfer catalyst, potassium ions, and water can be controlled.

In addition to the above-described heating operation, the heating process may involve an operation in which helium gas is introduced from the helium cylinder 22 via the valve 26f into the reaction vessel 16. In this case, the synthesizer 1 closes the three-way valve 26d to close the passage between the reaction vessel 16 and the reagent vessels 13 to 15 and the like, opens the valve 26g while opening the valve 26f to feed helium gas to the reaction vessel 16, and starts to heat the reaction vessel 16 using the heater 21. After that, the synthesizer 1 stops the heating by the heater 21 at an evaporation finish timing determined by the same method as above, closes the valve 26f to stop the feed of helium gas to the reaction vessel 16, and further closes the valve 26g to stop the evaporation process.

When the evaporation process is finished, the controller 30 reads and executes the synthesis module 39, and the synthesizer 1 synthesizes the target, $^{18}$F-FDG. First, the synthesizer 1 opens the three-way valves 26c and 26d to open the passage between the reagent vessel 14 and the reaction vessel 16. The synthesizer 1 then applies pressure to the reagent vessel 14 using the helium cylinder 22, thereby introducing the TATM solution of the reagent vessel 14 into the reaction vessel 16. When the introduction of the TATM solution into the reaction vessel 16 is finished, the synthesizer 1 finishes the pressurization with helium gas, and closes the three-way valve 26d to close the passage from the reaction vessel 16 to the reagent vessels 11 to 15. The synthesizer 1 then closes the valve 26g, uses the heater 21 to heat the reaction solution to give reaction conditions thereto, and synthesizes $^{18}$F-TAFDG by nucleophilic substitution reaction. The synthesizer 1 opens the valve 26g, and heats the reaction vessel 16 further to substantially evaporate the solvent from the reaction solution. The synthesizer 1 then opens the three-way valves 26d and 26e and applies pressure to the reagent vessel 15 using the helium cylinder 22 to introduce the hydrochloric acid of the reagent vessel 15 into the reaction vessel 16. The synthesizer 1 closes the valve 26g and the three-way valve 26d to seal the reaction vessel 16 again, and heats the reaction vessel 16 using the heater 21 to carry out acid hydrolysis. As the reaction conditions and amount of reagents in the above processes can be used known conditions (e.g. methods described in documents (Radioisotopes, 50 (2001), pp. 205-227; Radioisotopes, 50 (2001), pp. 228-256; and Production and quality control of radioactive agents for PET - A guideline to synthesis and clinical use (PET you houshasei yakuzai no seizou oyobi hinshitsu kanri - Gousei to rinshou shiyou heno tebiki), 2nd Edition, edited by PET Kagaku Workshop)).

In addition to the above-described heating operation, the above process of evaporating the solvent may involve an operation in which helium gas is introduced from the helium cylinder 22 via the valve 26f into the reaction vessel 16. In this case, after finishing the synthesis of $^{18}$F-TAFDG, the synthesizer 1 opens the valve 26g while opening the valve 26f to feed helium gas to the reaction vessel 16, and substantially evaporates the solvent from the reaction solution in the same manner as above. Then, after introducing the hydrochloric acid of the reagent vessel 15 into the reaction vessel 16 in the same operation as above, the synthesizer 1 closes the valves 26d, 26f, and 26g to seal the reaction vessel 16, and carries out acid hydrolysis in the same manner as above.

When the synthesis process is finished, the controller 30 reads and executes the purification module 40, thereby purifying the synthesized $^{18}$F-FDG. First, the synthesizer 1 opens the three-way valves 26d and 26e to open the passage between the reaction vessel 16 and the $^{18}$F-FDG recovery vessel 17. The synthesizer 1 opens the valve 26f and uses the helium cylinder 22 under this condition to apply pressure to the reaction vessel 16, thereby passing the reaction solution from the reaction vessel 16 through the purification column 18 to recover it in the $^{18}$F-FDG recovery vessel 17.

As the conditions and column to be used in the above processes can be used known conditions (e.g. methods described in documents (Radioisotopes, 50 (2001), pp. 205-227; Radioisotopes, 50 (2001), pp. 228-256; and Production and quality control of radioactive agents for PET - A guideline to synthesis and clinical use (PET you houshasei yakuzai no seizou oyobi hinshitsu kanri - Gousei to rinshou shiyou heno tebiki), 2nd Edition, edited by PET Kagaku Workshop)).

[Examples of calculating an evaporation finish timing]

Described here will be an example of detecting a particular time point and an example of calculating an evaporation finish timing based on a particular time point.

Figure 5 is a flowchart for detecting as a particular time point a point at which, during a period from a time when a trend in temperature changes from up to down to a time when the trend changes again to up, the negative gradient is maximum. The flowchart shown in Figure 5 shows one example of a process corresponding to the process of detecting a particular time point (S12) in the flowchart shown in Figure 3. First, the controller 30 determines a point at which the trend in temperature changes from up to down, i.e. a local maximum value of the change in temperature (S20). Then, the controller 30 detects a point at which the gradient is maximum (S22). Thus detecting a particular time point after the change in temperature shifted to a downward trend can prevent a false detection of a particular time point in an unstable change in temperature during an increase in temperature.

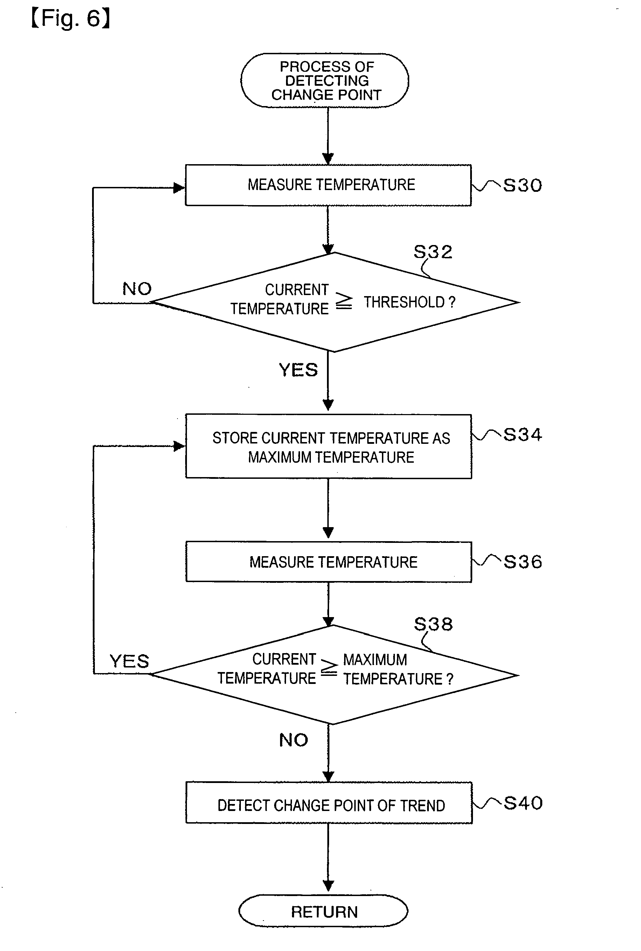

Figure 6 is a flowchart showing the process of detecting a point at which the temperature trend changes from up to down (S20). First, the controller 30 measures the temperature (S30), and determines whether or not the measured temperature (hereinafter referred to as the "current temperature") has reached a prescribed temperature (hereinafter referred to as the "threshold") or above (S32). The threshold to be used for the determination differs depending on the heating temperature to be used for the evaporation and the position to measure the temperature. For example, in a case where the heating temperature in the evaporation process is 110 degrees C and the thermometer 25 is located 5 mm from the reaction vessel 16, the threshold is set to approximately an initial temperature plus 15 degrees C. When it is evident that the noise of the curve indicating the change in temperature with time is small and that the temperature increases monotonously to a point at which the trend in temperature changes from up to down, the processes of the steps S30 and S32 may be omitted and an initial temperature may be stored as the maximum temperature.

If the measured temperature is lower than the threshold (NO at S32), the procedure returns to the step of measuring the temperature (S30). In the above configuration where the procedure does not move to the next step until the current temperature reaches a threshold or above, a false detection can be prevented that is caused by an unstable change in temperature during an increase in temperature, and the load on the process can be reduced. If the measured temperature is equal to or higher than the threshold (YES at S32), the controller 30 stores the current temperature as the maximum temperature (S34).

The controller 30 then measures the temperature (S36), and compares the measured temperature and the stored maximum temperature (S38). If the current temperature is equal to or higher than the stored maximum temperature (YES at S38), the controller 30 updates the maximum temperature with the current temperature to store it (S34), and measures the temperature again (S36). If the current temperature is lower than the stored maximum temperature (NO at S38), the controller 30 detects the time as a change point at which the trend changes from up to down (S40), and stores data indicating the maximum temperature stored at that point (temperature and time) as data on the change point. The process of detecting a change point (S20) is completed with the above processes.

In a case where the noise of temperature measurement data is large, temperature measurement data may be smoothed in advance, or the processes of the steps S34, S36, and S38 may be repeated until a current temperature indicates a value less than the maximum temperature a plurality of times in succession (e.g. until a current temperature lower than the maximum temperature is measured two times in succession).

When the process of detecting a change point (S20) is finished, the process of detecting a maximum negative gradient is performed (S22) as shown in Figure 5.

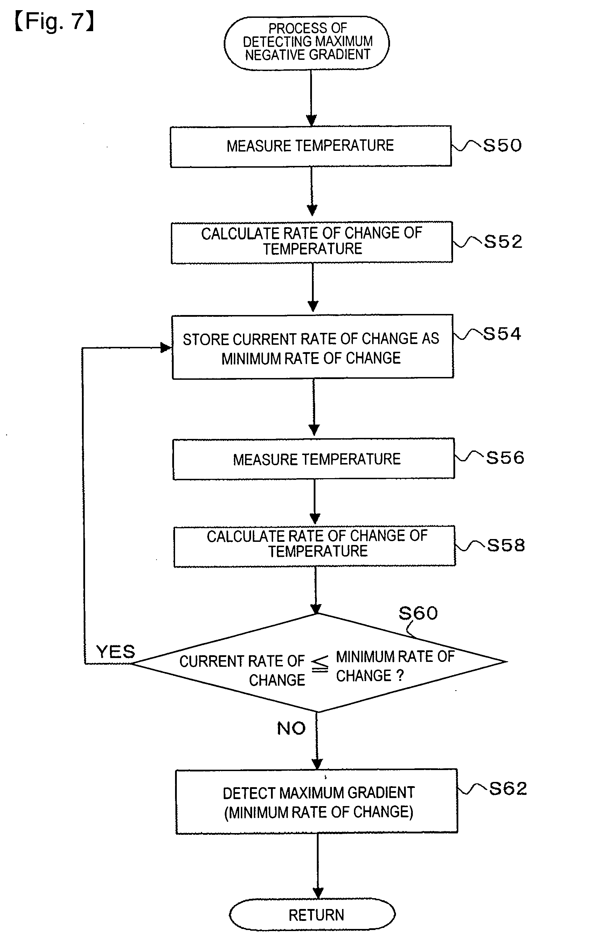

Figure 7 is a flowchart showing the process of detecting a maximum negative gradient (S22) in detail. As shown in Figure 7, in the process of detecting a maximum negative gradient, the controller 30 first measures the temperature (S50), and calculates a rate of change of the measured temperature (S52). For example, let $T_1$ and $T_2$ be values of temperature measured at successive measurement times $t_1$ and $t_2$, respectively, and then the rate of change of the temperature can be determined from Equation (1):

$$\text{Rate of change} = \frac{T_2 - T_1}{t_2 - t_1} \quad (1)$$

The controller 30 stores a first rate-of-change value determined from the above calculation, as the minimum rate-of-change value (S54). Then, the controller 30 measures the temperature (S56), and calculates the rate of change of the measured temperature (S58). The controller 30 compares the calculated rate-of-change value and the stored minimum rate-of-change value (S60). If the calculated rate of change is equal to or less than the stored rate of change (YES at S60), which is a case where a current negative gradient is equal to or larger than a stored negative gradient, then the controller 30 stores the current rate of change of the temperature as the minimum rate-of-change value (S54), and again moves to the step of measuring the temperature (S56). The controller 30 repeats the processes of measuring the temperature (S56), calculating the rate of change of the temperature (S58), and comparing (S60) until a calculated rate of change exceeds the stored minimum rate-of-change value.

If the calculated rate of change is larger than the stored minimum rate-of-change value (NO at S60), which is a case where a current negative gradient is less than a stored negative gradient, then the controller 30 detects the maximum negative gradient (S62), and stores the time at which the stored minimum value was measured, as the time of detection of a maximum negative gradient. The process of detecting a maximum negative gradient (S22) is completed with the above series of processes, and a time at which a maximum negative gradient appears in a downward trend can be detected as a particular time point.

In a case where the noise of temperature measurement data is large, the controller 30 may smooth temperature measurement data in advance, or may repeat the processes of the steps (S54, S56, and S58) until a rate of change indicates a value larger than the stored minimum rate-of-change value a plurality of times in succession.

In a case where the temperature is measured at a constant time interval, the rate of change calculated from the above Equation (1) may be replaced with the amount of change in temperature calculated from the following Equation (2):

$$\Delta T = T_2 - T_1 \quad (2)$$

Another example of detecting a particular time point will be described next.

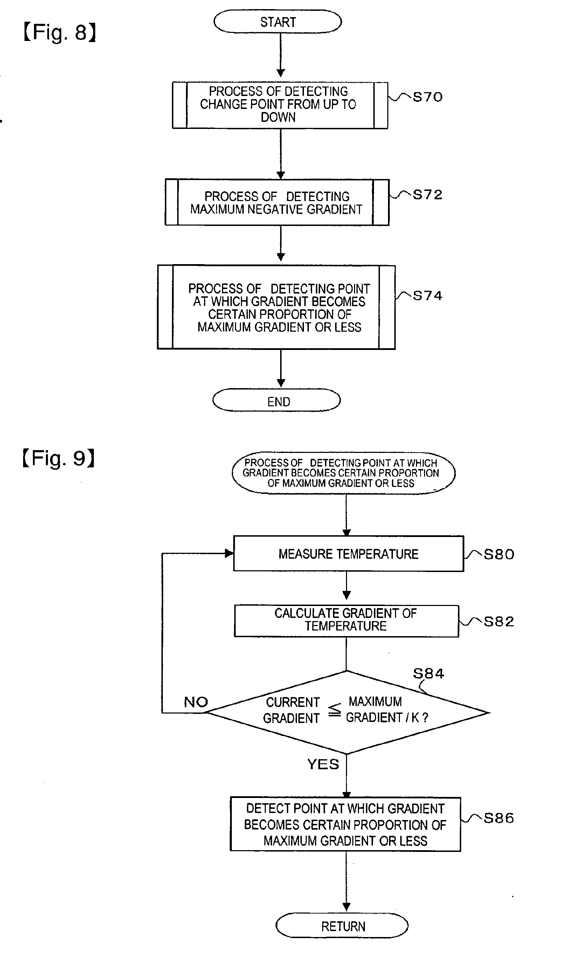

Figure 8 is a flowchart for, with reference to a point at which, during a period from a time when a trend in temperature changes from up to down to a time when the trend changes again to up, the negative gradient is maximum, detecting as a particular time point a point at which the gradient becomes a certain proportion of the maximum negative gradient or less. First, the controller 30 determines a point at which the change in temperature changes from an upward trend to a downward trend, i.e. a local maximum value of the change in temperature (S70). Then, the controller 30 detects a point at which the negative gradient is maximum (S72). The processes so far are the same as the flowchart shown in Figure 5.

The controller 30 then performs a process of detecting a point at which the negative gradient becomes a certain proportion (e.g. 1/10) of the maximum negative gradient or less (S74).

Figure 9 is a flowchart showing the process of detecting a particular time point at which a current gradient becomes a certain proportion of the maximum negative gradient or less. First, the controller 30 measures the temperature (S80), and calculates a rate of change of the temperature (S82). Then, the controller 30 compares the absolute value of the calculated rate of change of the temperature (which indicates the negative gradient) and the absolute value of the maximum gradient (which indicates the maximum negative gradient) divided by a prescribed number that is larger than one, K (K=10 if the certain proportion is 1/10) (S84). After the above determination, if a current gradient is not the certain proportion of the maximum gradient or less (NO at S84), the controller 30 again returns to the step of measuring the temperature (S80). If a current gradient is the certain proportion of the maximum gradient or less (YES at S84), the controller 30 detects the point at which the current gradient became the certain proportion of the maximum negative gradient (S86), and stores its time as the time of detection. The process of detecting a point at which a current gradient becomes a certain proportion of the maximum negative gradient or less (S74) is completed with the above series of processes.

In the above example, a particular time point at which the trend changes is determined by focusing on a maximum negative gradient in a downward trend. Alternatively, a point at which the first derivative of a temperature measurement result becomes zero may be found as a particular time point. This allows to detect a particular time point at which the trend in temperature changes from down to up. A point at which the second derivative of a temperature measurement result becomes zero may also be found as a particular time point. For example, if a particular time point at which the second derivative becomes zero is found when the change in temperature is in a downward trend, substantially the same point as the maximum negative gradient can be detected.

A method of determining a finish timing of the evaporation from a change point of the trend will be described next. In the embodiment, a time of detection of a change point of the trend added with a prescribed time is determined as a finish timing of the evaporation. The value to be added here is determined such that a preferred amount of water remains in the reaction solution after the evaporation process is finished. Specifically, the value to be added to a time of detection of a particular time point differs depending on the type of a used change point and on the heating temperature for the reaction vessel 16 in the evaporation process. Those skilled in the art could determine the value to be added on a daily basis by examining a change with time in the amount of water remaining after the evaporation process performed under various conditions, as described in the working examples below.

For example, in a case where a time at which a maximum negative gradient appears is used as a prescribed point at which the trend changes, if the heating temperature is 110 to 120 degrees C, an appropriate time within the range of 0 to 300 seconds can be selected as the value to be added, and the range of 60 to 240 seconds is preferable. If the heating temperature is 105 degrees C, the range of 100 to 300 seconds is preferable.

In a case where a time at which a change point at which the trend changes from down to up appears is used as a prescribed point at which the trend changes, if the heating temperature is 110 to 120 degrees C, an appropriate time within the range of 0 to 240 seconds can be selected as the value to be added, and the range of 60 to 180 seconds is preferable. If the heating temperature is 105 degrees C, the range of 10 to 180 seconds is preferable.

Up to this point, there have been described a synthesizer, program, and method of producing a radioactive-fluorine-labeled compound of the embodiment of the invention.

The synthesizer 1 of the above-described embodiment has the thermometer 25 for measuring a temperature of an outer wall of the outlet tube 23 for discharging evaporated water from the reaction vessel 16 and measures a temperature of the outlet tube 23 during the evaporation process, thereby being able to monitor the progress of the evaporation process.

The synthesizer 1 of the above-described embodiment also has the controller 30 that finishes the evaporation process based on the change in temperature measured by the thermometer 25, and finishes the evaporation with a prescribed amount of water remaining by means of the controller 30. A high radioactive fluorination yield can be achieved by producing $^{18}$F-FDG from a reaction solution including [$^{18}$F] fluoride ions, potassium carbonate, and a phase transfer catalyst, with a prescribed amount of residual water.

In the above embodiment, there has been described an example where the synthesizer 1, during the evaporation process, detects a particular time point at which the trend in temperature of an outer wall of the outlet tube 23 changes (S12) and determines a finish timing of the evaporation process based on the particular time point (S14). Alternatively, a finish timing of the evaporation process can be determined in advance. The synthesizer 1 produces $^{18}$F-FDG in advance, and then the evaporation finish timing is determined by the same method as the above-described embodiment and stored. When $^{18}$F-FDG is produced next under the same production conditions, the evaporation process is finished at the stored evaporation finish timing. Consequently, an evaporation finish timing need not be determined each time when $^{18}$F-FDG is produced, and therefore the calculation process can be simplified. The method in which the evaporation finish timing is determined in advance in this way is suitable for industrially producing $^{18}$F-FDG, or the like. The synthesizer 1 may store a plurality of evaporation finish timings in accordance with the production conditions so that the evaporation process is finished at an evaporation finish timing corresponding to the production conditions.

EXAMPLES

Now, the invention will be described in more detail with working examples and reference examples, but the invention is not limited to the following.

(Examples 1 to 9 and Comparative Examples 1 and 2) The relation between time elapsed from a time at which the maximum negative gradient appeared and water content of a sample

In a 5 mL vial were mixed 0.3 mL of water, 0.3 mL of a 66 mmol/L potassium carbonate solution, and a solution of 20 mg of Kryptofix 222 (product name, manufactured by MERCK) dissolved in 1.5 mL of acetonitrile. This vial was plugged, and then connected with a helium introduction tube and an outlet tube (made of PEEK, 0.75 mm in inner diameter, and 100 mm in length). A thermometer (sensor: K-type) and a temperature recorder were attached onto the above outlet tube at a position 5 mm away from the connection with the above vial.

With helium gas being introduced (flow rate: 50 mL/min), the above vial was heated to 110 degrees C by an air heater, and the change in temperature on the above outlet tube with time was measured by the above thermometer at one-second intervals. A time at which the maximum negative gradient appeared on the curve of the change in temperature with time was determined by the procedure described with Figures 5 to 7, and the evaporation process was finished after the time described in Table 1 had elapsed from the time concerned.

[Table 1]

(Table 1)

| | Time elapsed from maximum-negative-gradient time to evaporation finish timing (s) |
|---|---|
| Example 1 | 40 |
| Example 2 | 55 |
| Example 3 | 60 |

(continued)

| | Time elapsed from maximum-negative-gradient time to evaporation finish timing (s) |
|---|---|
| Example 4 | 95 |
| Example 5 | 100 |
| Example 6 | 135 |
| Example 7 | 150 |
| Example 8 | 210 |
| Example 9 | 250 |
| Comparative Example 1 | 335 |
| Comparative Example 2 | 350 |

The sample in the reaction vessel was cooled to room temperature, and the amount of water was measured by gas chromatography under the following conditions.

(Gas chromatography conditions)

Column: HP-INNOWax (product name, manufactured by Agilent Technologies, 0.53 mm in inner diameter, and 15 m in length)
Detector: TCD
Injector injection volume: 1 $\mu$L
Inlet heater temperature: 200 degrees C
Split ratio: 5:1
Column temperature: 60 degrees C
Detector heater temperature: 200 degrees C

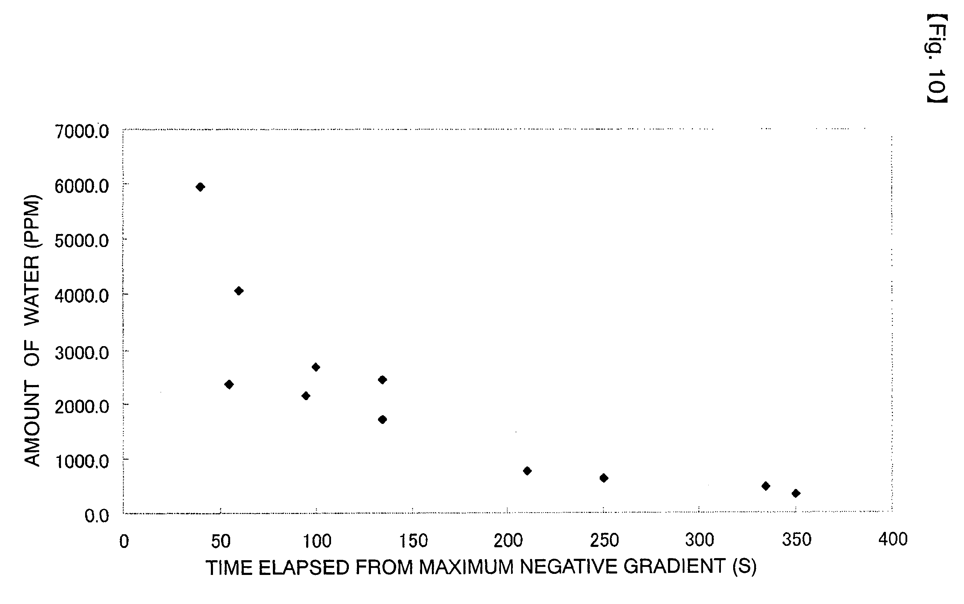

The result is shown in Table 2 and Figure 10. As shown in Table 2 and Figure 10, when the evaporation was finished after 40 to 250 seconds had elapsed from a time point at which the maximum negative gradient appeared (Examples 1 to 9), the amounts of water in the samples were 634.4 to 5948.1 (ppm). The inventors' study revealed that water content of the reaction solution was preferably 500 to 10000 (ppm) for producing $^{18}$F-FDG (and $^{18}$F-TAFDG) at a high yield (Japanese Patent Application No. 2005-352464). The amounts of water in the samples prepared under the conditions of Examples 1 to 9 all fell within the above range, and it was suggested that the yield of $^{18}$F-FDG could be improved by preparing the reaction solution using the above conditions in producing $^{18}$F-FDG.

[Table 2]

(Table 2) The relation between time elapsed from a time point at which the maximum negative gradient appeared and water content of the sample

| | Time elapsed from maximum-negative-gradient time to evaporation finish timing (s) | Water content (ppm) |
|---|---|---|
| Example 1 | 40 | 5948.1 |
| Example 2 | 55 | 2385.9 |
| Example 3 | 60 | 4054.9 |
| Example 4 | 95 | 2157.0 |
| Example 5 | 100 | 2684.2 |
| Example 6 | 135 | 1696.1 |
| Example 7 | 150 | 2445.2 |
| Example 8 | 210 | 766.8 |

(continued)

|  | Time elapsed from maximum-negative-gradient time to evaporation finish timing (s) | Water content (ppm) |
|---|---|---|
| Example 9 | 250 | 634.4 |
| Comparative Example 1 | 335 | 461.4 |
| Comparative Example 2 | 350 | 344.4 |

(Examples 10 to 14 and Comparative Example 3) The relation between time elapsed from a time at which the absolute value of the gradient indicated 1/10 of the maximum negative gradient and water content of a sample

The same process was performed and the same sample was prepared as Examples 1 to 9 except that a time at which the absolute value of the gradient of the curve of the change in temperature with time indicated 1/10 of the maximum negative gradient was determined by the procedure described with the above Figures 8 and 9, and the evaporation process was finished after the time described in Table 3 had elapsed from the time concerned.

[Table 3]

(Table 3) Time elapsed from a time point at which 1/10 of the maximum negative gradient appeared

|  | Time elapsed from time at which 1 /10 of maximum negative gradient appeared to evaporation finish timing (s) |
|---|---|
| Example 10 | 10 |
| Example 11 | 30 |
| Example 12 | 60 |
| Example 13 | 90 |
| Example 14 | 180 |
| Comparative Example 3 | 300 |

The sample in the reaction vessel was cooled to room temperature, and the amount of water was measured by gas chromatography under the same conditions as Examples 1 to 9. Each sample preparation and measurement were repeated twice.

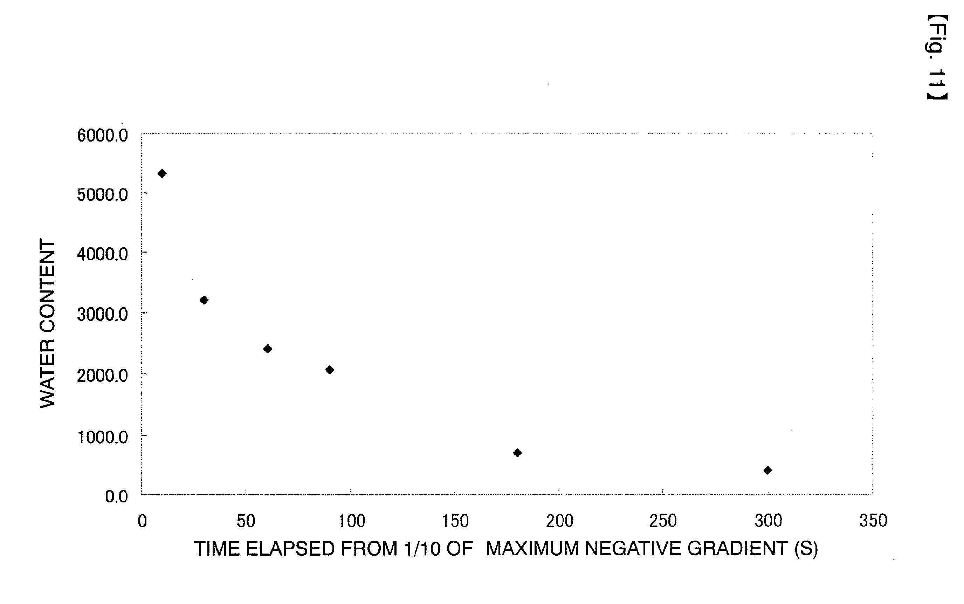

The result is shown in Table 4 and Figure 11. As shown in Table 4 and Figure 11, it was confirmed that the samples prepared under the conditions of Examples 10 to 14 all contained preferable water (500 to 10000 ppm) for producing $^{18}$F-FDG (and $^{18}$F-TAFDG) at a high yield. It was thus suggested that the yield of $^{18}$F-FDG could be improved by preparing the reaction solution using at least the conditions shown in Examples 10 to 14 in producing $^{18}$F-FDG.

[Table 4]

(Table 4) The relation between time elapsed from a time point at which 1/10 of the maximum negative gradient appeared and water content of the sample

|  | Time elapsed from time at which 1/10 of maximum negative gradient appeared to evaporation finish timing (s) | Water content (ppm) |
|---|---|---|
| Example 10 | 10 | 5314.4 |
| Example 11 | 30 | 3206.9 |

(continued)

| | Time elapsed from time at which 1/10 of maximum negative gradient appeared to evaporation finish timing (s) | Water content (ppm) |
|---|---|---|
| Example 12 | 60 | 2420.6 |
| Example 13 | 90 | 2070.7 |
| Example 14 | 180 | 700.6 |
| Comparative Example 3 | 300 | 402.9 |

(Examples 15 and 16) The relation between time elapsed from a time at which the absolute value of the gradient indicated 1/10 of the maximum negative gradient and water content of a sample

The same process was performed and the same sample was prepared as Examples 10 to 14 except that the heating temperature of the vial was 105 degrees C and that the evaporation process was finished after the time described in Table 5 had elapsed from a time at which 1/10 of the maximum negative gradient appeared.

[Table 5]

(Table 5) Time elapsed from a time point at which 1/10 of the maximum negative gradient appeared

| | Time elapsed from time at which 1/10 of maximum negative gradient appeared to evaporation finish timing (s) |
|---|---|
| Example 15 | 10 |
| Example 16 | 180 |

The sample in the reaction vessel was cooled to room temperature, and the amount of water was measured by gas chromatography under the same conditions as Examples 1 to 14.

The result is shown in Table 6. As shown in Table 6, it was confirmed that the samples prepared under the conditions of Examples 15 and 16 both contained preferable water (500 to 10000 ppm) for producing $^{18}$F-FDG (and $^{18}$F-TAFDG) at a high yield. It was thus suggested that the yield of $^{18}$F-FDG could be improved by preparing the reaction solution using the above conditions in producing $^{18}$F-FDG.

[Table 6]

(Table 6) The relation between time elapsed from a time point at which 1/10 of the maximum negative gradient appeared and water content of the sample

| | Time elapsed from time at which 1/10 of maximum negative gradient appeared to evaporation finish timing (s) | Water content (ppm) |
|---|---|---|
| Example 15 | 10 | 5935.7 |
| Example 16 | 180 | 1895.2 |

(Examples 17 and 18) The relation between time elapsed from a time at which the absolute value of the gradient indicated 1/10 of the maximum negative gradient and water content of a sample

The same process was performed and the same sample was prepared as Examples 10 to 14 except that the heating temperature of the vial was 120 degrees C and that the evaporation process was finished after the time described in Table 7 had elapsed from a time at which 1/10 of the maximum negative gradient appeared.

[Table 7]

(Table 7) Time elapsed from a time point at which 1/10 of the maximum negative gradient appeared

|  | Time elapsed from time at which 1/10 of maximum negative gradient appeared to evaporation finish timing (s) |
|---|---|
| Example 17 | 10 |
| Example 18 | 180 |

The sample in the reaction vessel was cooled to room temperature, and the amount of water was measured by gas chromatography under the same conditions as Examples 1 to 16.

The result is shown in Table 8. As shown in Table 8, it was confirmed that the samples prepared under the conditions of Examples 17 and 18 both contained preferable water (500 to 10000 ppm) for producing $^{18}$F-FDG (and $^{18}$F-TAFDG) at a high yield. It was thus suggested that the yield of $^{18}$F-FDG could be improved by preparing the reaction solution using the above conditions in producing $^{18}$F-FDG.

[Table 8]

(Table 8) The relation between time elapsed from a time point at which 1/10 of the maximum negative gradient appeared and water content of the sample

|  | Time elapsed from time at which 1/10 of maximum negative gradient appeared to evaporation finish timing (s) | Water content (ppm) |
|---|---|---|
| Example 17 | 10 | 6219.9 |
| Example 18 | 180 | 975.3 |

(Examples 19 and 20) The relation between time elapsed from a time at which the absolute value of the gradient indicated 1/10 of the maximum negative gradient and water content of a sample

The same process was performed and the same sample was prepared as Examples 10 to 14 except that the thermometer was placed on the outlet tube at a position 14 mm from the top surface of the vial and that the evaporation process was finished after the time described in Table 9 had elapsed from a time at which 1/10 of the maximum negative gradient appeared.

[Table 9]

(Table 9) Time elapsed from a time point at which 1/10 of the maximum negative gradient appeared

|  | Time elapsed from time at which 1/10 of maximum negative gradient appeared to evaporation finish timing (s) |
|---|---|
| Example 19 | 10 |
| Example 20 | 180 |

The sample in the reaction vessel was cooled to room temperature, and the amount of water was measured by gas chromatography under the same conditions as Examples 1 to 18.

The result is shown in Table 10. As shown in Table 10, it was confirmed that the samples prepared under the conditions of Examples 19 and 20 both contained preferable water (500 to 10000 ppm) for producing [18F]-FDG (and [18F]-TAFDG) at a high yield. It was thus suggested that the yield of [18F]-FDG could be improved by preparing the reaction solution using the above conditions in producing [18F]-FDG.

[Table 10]

(Table 10) The relation between time elapsed from a time point at which 1/10 of the maximum negative gradient appeared and water content of the sample

| | Time elapsed from time at which 1/10 of maximum negative gradient appeared to evaporation finish timing (s) | Water content (ppm) |
| --- | --- | --- |
| Example 19 | 10 | 4352.6 |
| Example 20 | 180 | 656.3 |

(Examples 21 to 23) The production yield of [18F]-FDG in the present method

Target water enriched with [18O] was subjected to proton radiation to obtain [18F] fluoride ions as [18F]-fluoride-ions-containing target water. Radioactivity associated with this [18F]-fluoride-ions-containing target water was measured by CRC-15R (product name, manufactured by CAPINTEC, INC.) (which is referred to as the radioactivity A), and then the [18F]-fluoride-ions-containing target water was passed through an anion exchange column to adsorb and collect [18F] fluoride ions, through which a potassium carbonate solution was passed to elute the [18F] fluoride ions in a reaction vessel. This eluate including [18F] fluoride ions was added with an acetonitrile solution of Kryptofix 222 (product name, manufactured by MERCK). The amount and addition method of the potassium carbonate and phase transfer catalyst followed usual methods (methods described in: Radioisotopes, 50 (2001), pp. 205-227; Radioisotopes, 50 (2001), pp. 228-256; and Production and quality control of radioactive agents for PET - A guideline to synthesis and clinical use (PET you houshasei yakuzai no seizou oyobi hinshitsu kanri - Gousei to rinshou shiyou heno tebiki), 2nd Edition, edited by PET Kagaku Workshop).

The above reaction vessel was connected with a helium introduction tube and an outlet tube, and a thermometer (sensor: K-type) and a temperature recorder were connected onto the outlet tube concerned at a position 4 cm from the reaction vessel. With helium gas being introduced (flow rate: 50 mL/min), the above reaction vessel was heated to 110 degrees C by an air heater, and the change in temperature on the above outlet tube with time was measured by the above thermometer at one-second intervals. A time at which the absolute value of the gradient of the curve of the change in temperature with time indicated 1/10 of the maximum negative gradient was determined by performing the procedure described with the above Figures 8 and 9, and the evaporation process was finished after the time described in Table 11 had elapsed from the time concerned.

[Table 11]

(Table 11) Time elapsed from a time point at which 1/10 of the maximum negative gradient appeared

| | Time elapsed from time at which 1/10 of maximum negative gradient appeared to evaporation finish timing (s) |
| --- | --- |
| Example 21 | 45 |
| Example 22 | 75 |
| Example 23 | 90 |

After the evaporation process was finished, the reaction vessel was added with 1 mL of an acetonitrile solution of TATM (concentration: 20 mg/mL) to prepare a reaction solution. Then, the reaction vessel was plugged; a labeling reaction was performed by heating with an air heater; and with helium gas being introduced (flow rate: 50 mL/min) with the vessel opened, the solvent was evaporated by further heating. The conditions applied here followed usual methods (methods described in: Radioisotopes, 50 (2001), pp. 205-227; Radioisotopes, 50 (2001), pp. 228-256; and Production and quality control of radioactive agents for PET - A guideline to synthesis and clinical use (PET you houshasei yakuzai no seizou oyobi hinshitsu kanri - Gousei to rinshou shiyou heno tebiki), 2nd Edition, edited by PET Kagaku Workshop).

Acid hydrolysis was carried out by following usual methods (methods described in: Radioisotopes, 50 (2001), pp. 205-227; Radioisotopes, 50 (2001), pp. 228-256; and Production and quality control of radioactive agents for PET - A guideline to synthesis and clinical use (PET you houshasei yakuzai no seizou oyobi hinshitsu kanri - Gousei to rinshou shiyou heno tebiki), 2nd Edition, edited by PET Kagaku Workshop), and the obtained solution was further subjected to column purification according to usual methods (methods described in: Radioisotopes, 50 (2001), pp. 205-227; Radioisotopes, 50 (2001), pp. 228-256; and Production and quality control of radioactive agents for PET - A guideline to synthesis and clinical use (PET you houshasei yakuzai no seizou oyobi hinshitsu kanri - Gousei to rinshou shiyou heno tebiki), 2nd Edition, edited by PET Kagaku Workshop) to obtain an $^{18}$F-FDG solution. Radioactivity associated with the obtained $^{18}$F-FDG solution was measured by CRC-15R (product name, manufactured by CAPINTEC, INC.) (the obtained radioactivity is referred to as B), and the yield was determined from the following Equation (3):

$$\text{Yield}\,(\%) = \frac{\text{B}}{\text{A}} \times 100 \qquad (3)$$

The result is shown in Table 12. As shown in Table 12, $^{18}$F-FDG was able to be obtained at a yield of 75% or higher under any of the conditions of Examples 21 to 23. This result indicated that $^{18}$F-FDG could be produced at a high yield by the method according to the invention.

[Table 12]

(Table 12) The relation between time elapsed from a time point at which 1/10 of the maximum negative gradient appeared and the yield

|  | Time elapsed from time at which 1/10 of maximum negative gradient appeared to evaporation finish timing (s) | Yield (%) |
|---|---|---|
| Example 21 | 45 | 77.4 |
| Example 22 | 75 | 78.0 |
| Example 23 | 90 | 80.9 |

(Examples 24 to 33) The relation between a measurement point and a particular time point

In a 5 mL vial were mixed 0.3 mL of water, 0.3 mL of a 66 mmol/L potassium carbonate solution, and a solution of 20 mg of Kryptofix 222 (product name, manufactured by MERCK) dissolved in 1.5 mL of acetonitrile. This vial was plugged, and then connected with an outlet tube (made of PEEK, 0.75 mm in inner diameter, and 100 mm in length). A thermometer (sensor: K-type) and a temperature recorder were attached onto the above outlet tube at a position a distance described in Table 13 away from the connection with the above vial.

[Table 13]

(Table 13)

|  | Attachment position of temperature sensor on outlet tube (Distance from vial, mm) |
|---|---|
| Example 24 | 5 |

(continued)

| | Attachment position of temperature sensor on outlet tube (Distance from vial, mm) |
|---|---|
| Example 25 | 25 |
| Example 26 | 50 |
| Example 27 | 75 |
| Example 28 | 100 |
| Example 29 | 150 |
| Example 30 | 200 |
| Example 31 | 300 |
| Example 32 | 400 |
| Example 33 | 500 |

The above vial was heated to 110 degrees C by an air heater, and the change in temperature on the above outlet tube with time was measured by the above thermometer at one-second intervals. A time at which the maximum negative gradient appeared on the curve of the change in temperature with time was determined by the procedure described with the above Figures 5 to 7.

The result is shown in Table 14. As shown in this table, the time at which the maximum negative gradient appeared was approximately constant regardless of measurement point. This result indicated that in a case where a time at which the maximum negative gradient appeared was the particular time point, the particular time point did not differ depending on the measurement point and any measurement point might be used in the range of 5 to 500 mm.

[Table 14]

(Table 14)

| | Attachment position of temperature sensor on outlet tube (Distance from vial, mm) | Particular time point that was time at which maximum negative gradient appeared (Time elapsed from start of measurement) |
|---|---|---|
| Example 24 | 5 | 10 min 49 s |
| Example 25 | 25 | 10 min 54 s |
| Example 26 | 50 | 10 min 49 s |
| Example 27 | 75 | 10 min 54 s |
| Example 28 | 100 | 10 min 54 s |
| Example 29 | 150 | 10 min 54 s |
| Example 30 | 200 | 10 min 54 s |
| Example 31 | 300 | 10 min 54 s |
| Example 32 | 400 | 10 min 54 s |
| Example 33 | 500 | 10 min 54 s |

While there have been described what are at present considered to be preferred embodiments of the invention, it will be understood that various modifications and variations may be made thereto, and it is intended that appended claims cover all such modifications and variations as fall within the true spirit and scope of the invention.

Industrial applicability

The production method, synthesizer, and program for a radioactive-fluorine-labeled compound according to the invention are useful as a production apparatus, production method, and the like for radiopharmaceuticals.

The contents of the documents referenced herein are incorporated herein by reference.

**Claims**

1. A method of producing a radioactive-fluorine-labeled compound, the method comprising the steps of:

    heating in a reaction vessel a mixture containing [18F] fluoride ions, a phase transfer catalyst, potassium ions, and water to evaporate water from the mixture;
    preparing a reaction solution including the mixture and a labeling precursor compound 1,3,4,6-tetra-O-acetyl-2-O-trifluoromethanesulfonyl-β-D-mannopyranose; and
    obtaining 1,3,4,6-tetra-O-acetyl-2-[18F]fluoro-2-deoxyglucose by giving reaction conditions to the reaction solution,

    wherein the step of evaporating water has a step of measuring a temperature of an outlet tube for discharging evaporated water from the reaction vessel to determine a finish timing of the evaporation based on the measured temperature, and the evaporation is finished at the finish timing.

2. The method of producing a radioactive-fluorine-labeled compound according to claim 1, wherein in the step of determining a finish timing of the evaporation, a temperature of an outer wall of the outlet tube is measured.

3. The method of producing a radioactive-fluorine-labeled compound according to claim 1, wherein in the step of determining a finish timing of the evaporation, a finish timing of the evaporation is determined based on a change point at which a trend in the measured temperature, after changed from up to down, changes again to up.

4. The method of producing a radioactive-fluorine-labeled compound according to claim 1, wherein in the step of determining a finish timing of the evaporation, a finish timing of the evaporation is determined based on a point at which, during a period from a time when a trend in the measured temperature changes from up to down to a time when the trend changes again to up, the negative gradient of the change in temperature is maximum.

5. A method of producing a radioactive-fluorine-labeled compound, the method comprising the steps of:

    heating in a reaction vessel a mixture containing [18F] fluoride ions, a phase transfer catalyst, potassium ions, and water to evaporate water from the mixture;
    preparing a reaction solution including the mixture and a labeling precursor compound 1,3,4,6-tetra-O-acetyl-2-O-trifluoromethanesulfonyl-β-D-mannopyranose; and
    obtaining 1,3,4,6-tetra-O-acetyl-2-[18F]fluoro-2-deoxyglucose by giving reaction conditions to the reaction solution,

    wherein, in the step of evaporating water, the evaporation is finished at a predetermined evaporation finish timing, and wherein the evaporation finish timing is determined by measuring a temperature of an outlet tube for discharging evaporated water from the reaction vessel and being based on the measured temperature.

6. A method of producing a radioactive-fluorine-labeled compound, the method comprising the steps of:

    heating in a reaction vessel a mixture containing [18F] fluoride ions, a phase transfer catalyst, potassium ions, and water to evaporate water from the mixture;
    preparing a reaction solution including the mixture and a labeling precursor compound 1,3,4,6-tetra-O-acetyl-2-O-trifluoromethanesulfonyl-β-D-mannopyranose; and
    obtaining 1,3,4,6-tetra-O-acetyl-2-[18F]fluoro-2-deoxyglucose by giving reaction conditions to the reaction solution,

    wherein the step of evaporating water has a step of measuring a temperature of a component connected to the reaction vessel to determine a finish timing of the evaporation based on the measured temperature, and the evaporation is finished at the finish timing.

7. A method of controlling water content of a mixture containing [18F] fluoride ions, a phase transfer catalyst, potassium ions, and water, the method comprising the steps of:

heating in a reaction vessel a mixture containing [$^{18}$F] fluoride ions, a phase transfer catalyst, potassium ions, and water to start evaporation of water from the mixture;
measuring a temperature of an outlet tube for discharging evaporated water from the reaction vessel to determine a finish timing of the evaporation based on the measured temperature; and
finishing the evaporation of water at the finish timing.

8. The method of controlling water content of the mixture according to claim 7,
wherein in the step of determining a finish timing of the evaporation, a temperature of an outer wall of the outlet tube is measured.

9. The method of controlling water content of the mixture according to claim 7,
wherein in the step of determining a finish timing of the evaporation, a finish timing of the evaporation is determined based on a change point at which a trend in the measured temperature, after changed from up to down, changes again to up.

10. The method of controlling water content of the mixture according to claim 7,
wherein in the step of determining a finish timing of the evaporation, a finish timing of the evaporation is determined based on a point at which, during a period from a time when a trend in the measured temperature changes from up to down to a time when the trend changes again to up, the negative gradient of the change in temperature is maximum.

11. A method of controlling water content of a mixture containing [$^{18}$F] fluoride ions, a phase transfer catalyst, potassium ions, and water, the method comprising the steps of:

heating in a reaction vessel a mixture containing [$^{18}$F] fluoride ions, a phase transfer catalyst, potassium ions, and water to start evaporation of water from the mixture; and
finishing the evaporation of water at a predetermined evaporation finish timing,

wherein the evaporation finish timing is determined by measuring a temperature of an outlet tube for discharging evaporated water from the reaction vessel and being based on the measured temperature.

12. A method of controlling water content of a mixture containing [$^{18}$F] fluoride ions, a phase transfer catalyst, potassium ions, and water, the method comprising the steps of:

heating in a reaction vessel a mixture containing [$^{18}$F] fluoride ions, a phase transfer catalyst, potassium ions, and water to start evaporation of water from the mixture;
measuring a temperature of a component connected to the reaction vessel to determine a finish timing of the evaporation based on the measured temperature; and
finishing the evaporation of water at the finish timing.

13. A synthesizer comprising:

a reaction vessel;
a heater for heating the reaction vessel in order to evaporate water from a mixture contained in the reaction vessel;
an outlet tube for discharging evaporated water from the reaction vessel; and
a thermometer for measuring a temperature of the outlet tube.

14. The synthesizer according to claim 13, wherein the thermometer measures a temperature of an outer wall of the outlet tube.

15. The synthesizer according to claim 13, wherein the thermometer is attached to the outlet tube at a position within 30 cm of a connection between the outlet tube and the reaction vessel.

16. The synthesizer according to claim 13, having a controller for controlling the heater based on a temperature measured by the thermometer.

17. The synthesizer according to claim 16, wherein the controller controls the heater such that water content of the mixture is within a prescribed range of values, based on a trend in temperature measured by the thermometer.

18. The synthesizer according to claim 16 for synthesizing $^{18}$F-FDG, wherein the controller controls the heater in such a way as to determine a finish timing to finish the evaporation based on a change point at which a trend in temperature measured by the thermometer, after changed from up to down, changes again to up, and as to finish the evaporation at the finish timing.

19. The synthesizer according to claim 16 for synthesizing $^{18}$F-FDG, wherein the controller controls the heater in such a way as to determine a finish timing to finish the evaporation based on a point at which, during a period from a time when a trend in temperature measured by the thermometer changes from up to down to a time when the trend changes again to up, the negative gradient of the change in temperature is maximum, and as to finish the evaporation at the finish timing.

20. A synthesizer comprising:

   a reaction vessel;
   a heater for heating the reaction vessel in order to evaporate water from a mixture contained in the reaction vessel;
   a thermometer for measuring a temperature of a component connected to the reaction vessel; and
   a controller for controlling the heater based on a temperature measured by the thermometer.

21. A program for evaporating water from a solution containing [$^{18}$F] fluoride ions, a phase transfer catalyst, and potassium ions by means of a synthesizer comprising: a reaction vessel; a heater for heating the reaction vessel in order to evaporate water from a solution contained in the reaction vessel; an outlet tube for discharging water evaporated from within the reaction vessel; and a thermometer for measuring a temperature of the outlet tube, the program causing the synthesizer to execute the steps of:

   heating the reaction vessel by means of the heater to start the evaporation;
   acquiring information on a temperature of the outlet tube from the thermometer;
   determining a finish timing of the evaporation based on the temperature; and
   finishing the evaporation at the finish timing.

22. The program according to claim 21, wherein in the step of determining a finish timing of the evaporation, a finish timing of the evaporation is determined based on a change point at which a trend in the acquired temperature, after changed from up to down, changes again to up.

23. The program according to claim 21, wherein in the step of determining a finish timing of the evaporation, a finish timing of the evaporation is determined based on a point at which, during a period from a time when a trend in the acquired temperature changes from up to down to a time when the trend changes again to up, the negative gradient of the change in temperature is maximum.

24. A program for evaporating water from a solution containing [$^{18}$F] fluoride ions, a phase transfer catalyst, and potassium ions by means of a synthesizer comprising: a reaction vessel; a heater for heating the reaction vessel in order to evaporate water from a solution contained in the reaction vessel; and a thermometer for measuring a temperature of a component connected to the reaction vessel, the program causing the synthesizer to execute the steps of:

   heating the reaction vessel by means of the heater to start the evaporation;
   acquiring information on a temperature of the component from the thermometer;
   determining a finish timing of the evaporation based on the temperature; and
   finishing the evaporation at the finish timing.

【Fig. 1】

【Fig. 2】

【Fig. 3】

```
        ┌─────────────────┐
        │      START      │
        └─────────────────┘
                 │
                 ▼
   ┌─────────────────────────────┐
   │  START  EVAPORATION PROCESS │  ～S10
   └─────────────────────────────┘
                 │
                 ▼
   ┌─────────────────────────────┐
   │   PROCESS OF DETECTING      │
   │   PRESCRIBED CHANGE IN      │
   │   TEMPERATURE TREND         │  ～S12
   │   (PARTICULAR TIME POINT)   │
   └─────────────────────────────┘
                 │
                 ▼
   ┌─────────────────────────────┐
   │   CALCULATE EVAPORATION     │
   │        FINISH TIME          │  ～S14
   └─────────────────────────────┘
                 │
                 ▼
   ┌─────────────────────────────┐
   │  FINISH EVAPORATION PROCESS │  ～S16
   └─────────────────────────────┘
                 │
                 ▼
        ┌─────────────────┐
        │       END       │
        └─────────────────┘
```

【Fig. 4】

TEMPERATURE TREND CONDITIONS FOR EACH MEASUREMENT POINT
(REACTION VESSEL: SV-5, EVAPORATION TEMPERATURE: 110 DEGREES C)

【Fig. 5】

```
        START
          │
          ▼
┌─────────────────────┐
│ PROCESS OF DETECTING│
│ CHANGE POINT FROM UP│ ～S20
│      TO DOWN        │
└─────────────────────┘
          │
          ▼
┌─────────────────────┐
│ PROCESS OF DETECTING│
│ MAXIMUM NEGATIVE    │ ～S22
│     GRADIENT        │
└─────────────────────┘
          │
          ▼
        RETURN
```

【Fig. 6】

```
        ┌─────────────────────┐
        │     PROCESS OF      │
        │     DETECTING       │
        │   CHANGE POINT      │
        └─────────────────────┘
                   │
                   ▼
        ┌─────────────────────┐
   ┌──▶│  MEASURE TEMPERATURE │──~S30
   │    └─────────────────────┘
   │               │
   │               ▼                    S32
   │          ╱─────────────────────────╲
   │  NO     ╱    CURRENT                 ╲
   ├────────◀      TEMPERATURE ≧ THRESHOLD?│
   │         ╲                           ╱
   │          ╲─────────────────────────╱
   │               │
   │              YES
   │               │
   │               ▼
   │    ┌──────────────────────────────┐
   │┌─▶│ STORE CURRENT TEMPERATURE AS   │──~S34
   ││   │    MAXIMUM TEMPERATURE         │
   ││   └──────────────────────────────┘
   ││              │
   ││              ▼
   ││   ┌──────────────────────────────┐
   ││   │     MEASURE TEMPERATURE        │──~S36
   ││   └──────────────────────────────┘
   ││              │
   ││              ▼                    S38
   ││         ╱─────────────────────────╲
   ││ YES    ╱  CURRENT    MAXIMUM       ╲
   │└───────◀  TEMPERATURE ≧ TEMPERATURE?│
   │         ╲                           ╱
   │          ╲─────────────────────────╱
   │               │
   │              NO
   │               │
   │               ▼
   │    ┌──────────────────────────────┐
   │    │ DETECT CHANGE POINT OF TREND   │──~S40
   │    └──────────────────────────────┘
   │               │
   │               ▼
   │        ┌─────────────────┐
   │        │     RETURN      │
   │        └─────────────────┘
```

【Fig. 7】

```
        ┌──────────────────────┐
        │     PROCESS OF        │
        │ DETECTING MAXIMUM     │
        │  NEGATIVE GRADIENT    │
        └──────────┬───────────┘
                   │
                   ▼
        ┌──────────────────────┐
        │  MEASURE TEMPERATURE  │─────S50
        └──────────┬───────────┘
                   │
                   ▼
        ┌──────────────────────┐
        │ CALCULATE RATE OF     │─────S52
        │ CHANGE OF TEMPERATURE │
        └──────────┬───────────┘
                   │
                   ▼
        ┌──────────────────────┐
        │ STORE CURRENT RATE    │─────S54
        │ OF CHANGE AS MINIMUM  │
        │ RATE OF CHANGE        │
        └──────────┬───────────┘
                   │
                   ▼
        ┌──────────────────────┐
        │  MEASURE TEMPERATURE  │─────S56
        └──────────┬───────────┘
                   │
                   ▼
        ┌──────────────────────┐
        │ CALCULATE RATE OF     │─────S58
        │ CHANGE OF TEMPERATURE │
        └──────────┬───────────┘
                   │
                   ▼
                  S60
        ◇──────────────────────◇
  YES   │ CURRENT RATE OF ≦     │
◄───────│ CHANGE   MINIMUM RATE │
        │ OF CHANGE ?           │
        ◇──────────┬───────────◇
                   │ NO
                   ▼
        ┌──────────────────────┐
        │ DETECT MAXIMUM        │─────S62
        │ GRADIENT (MINIMUM     │
        │ RATE OF CHANGE)       │
        └──────────┬───────────┘
                   │
                   ▼
        ┌──────────────────────┐
        │       RETURN          │
        └──────────────────────┘
```

【Fig. 8】

```
                    START

PROCESS OF DETECTING
CHANGE POINT FROM UP TO          S70
       DOWN

PROCESS OF  DETECTING            S72
MAXIMUM NEGATIVE GRADIENT

PROCESS OF  DETECTING POINT
AT WHICH GRADIENT BECOMES        S74
CERTAIN PROPORTION OF
MAXIMUM GRADIENT OR LESS

                     END
```

【Fig. 9】

```
PROCESS OF  DETECTING POINT AT WHICH
GRADIENT BECOMES CERTAIN PROPORTION
     OF MAXIMUM GRADIENT OR LESS

MEASURE TEMPERATURE               S80

CALCULATE GRADIENT OF             S82
     TEMPERATURE

                                  S84
NO      CURRENT    ≦  MAXIMUM
        GRADIENT      GRADIENT / K ?

              YES

DETECT POINT AT WHICH GRADIENT
BECOMES CERTAIN PROPORTION OF      S86
MAXIMUM GRADIENT OR LESS

              RETURN
```

【Fig. 10】

【Fig. 11】

<table>
<tr><td colspan="2">**INTERNATIONAL SEARCH REPORT**</td><td>International application No.<br>PCT/JP2007/067020</td></tr>
</table>

A. CLASSIFICATION OF SUBJECT MATTER
*C07H5/02*(2006.01)i, *C07B59/00*(2006.01)i, *G01T1/161*(2006.01)i, *C07B61/00* (2006.01)n

According to International Patent Classification (IPC) or to both national classification and IPC

B. FIELDS SEARCHED

Minimum documentation searched (classification system followed by classification symbols)
C07H5/02, C07B59/00, G01T1/161, C07B61/00

Documentation searched other than minimum documentation to the extent that such documents are included in the fields searched
Jitsuyo Shinan Koho            1922-1996   Jitsuyo Shinan Toroku Koho   1996-2007
Kokai Jitsuyo Shinan Koho      1971-2007   Toroku Jitsuyo Shinan Koho   1994-2007

Electronic data base consulted during the international search (name of data base and, where practicable, search terms used)
CAplus(STN)

C. DOCUMENTS CONSIDERED TO BE RELEVANT

| Category* | Citation of document, with indication, where appropriate, of the relevant passages | Relevant to claim No. |
|---|---|---|
| A | PADGETT, H. C. et al., Computer-controlled radiochemical synthesis: a chemistry process control unit for the automated production of radiochemicals, Applied Radiation and Isotopes, 1989, 40(5), p433-435,437-445(lack ofp436) | 1-24 |
| A | HAMACHER, Kurt et al., Efficient stereospecific synthesis of no-carrier-added 2-[18F]-fluoro-2-deoxy-D-glucose using aminopolyether supported nucleophilic substitution, Journal of Nuclear Medicine, 1986, 27(2), p235-238 | 1-24 |
| A | HAMACHER, K. et al., Electrochemical cell for separation of [18F]fluoride from irradiated 18O-water and subsequent no carrier added nucleophilic fluorination, Applied Radiation and Isotopes, 2002, 56(3), p519-523 | 1-24 |

☒  Further documents are listed in the continuation of Box C.        ☐  See patent family annex.

| | |
|---|---|
| * Special categories of cited documents:<br>"A"  document defining the general state of the art which is not considered   to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search<br>18 September, 2007 (18.09.07) | Date of mailing of the international search report<br>02 October, 2007 (02.10.07) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2007/067020

C (Continuation). DOCUMENTS CONSIDERED TO BE RELEVANT

| Category* | Citation of document, with indication, where appropriate, of the relevant passages | Relevant to claim No. |
|---|---|---|
| A | WO 2004/100490 A2 (SCHERING AG.), 18 November, 2004 (18.11.04), Full text & JP 2006-525279 A | 1-24 |
| A | US 2005/232861 A1 (BUCHANAN C R), 20 October, 2005 (20.10.05), Full text (Family: none) | 1-24 |
| A | JP 2005-520827 A (HAMMERSMITH IMANET LTD.), 14 July, 2005 (14.07.05), Full text & WO 2003/078358 A2 | 1-24 |
| A | JP 05-345731 A (NKK CORP.), 27 December, 1993 (27.12.93), Full text & JP 2738224 B2 | 1-24 |
| A | JP 06-157572 A (GENERAL ELECTRIC CO.), 03 June, 1994 (03.06.94), Full text & US 5264570 A | 1-24 |
| A | JP 09-263590 A (NKK CORP.), 07 October, 1997 (07.10.97), Full text & EP 798307 A1 | 1-24 |
| A | JP 10-219299 A (LION CORP.), 18 August, 1998 (18.08.98), Full text (Family: none) | 1-24 |
| P,A | WO 2007/063940 A1 (Nihon Medi-Physics Co., Ltd.), 07 June, 2007 (07.06.07), Full text (Family: none) | 1-24 |
| P,A | WO 2007/066567 A1 (Nihon Medi-Physics Co., Ltd.), 14 June, 2007 (14.06.07), Full text (Family: none) | 1-24 |

Form PCT/ISA/210 (continuation of second sheet) (April 2005)

| **INTERNATIONAL SEARCH REPORT** | International application No. |
|---|---|
| | PCT/JP2007/067020 |

With respect to claims 13-17 and 20, as these fail to describe for what purpose the "synthetic apparatus" is used, the apparatus is unclear. Further, it is unclear what of heater is controlled on the basis of temperature measurement results, and the relationship between the synthetic apparatus and the reactor vessel cannot be understood. Consequently, the invention of the apparatus in this application cannot be clearly comprehended.

Moreover, as simply "the temperature of the external wall of the emission pipe" or simply "the temperature of parts connected" to the reactor vessel cannot be interpreted as always reflecting the temperature of water evaporated off in dependence upon the type of material or fitting position thereof, what technical significance lies in the control of heater on the basis of the above temperature cannot be seen. Thus, the invention of the above claims is unclear.

Furthermore, with respect to claims 18-19, as the relationship between the synthetic apparatus for 18F-FDG and the heater interpreted as constituting part thereof cannot be seen, the invention of the apparatus in this application is unclear.

Therefore, in this search report, search has been focused on the invention relating to the technology of, in the evaporation-off of water from a mixture containing 18F fluoride ions, a phase-transfer catalyst, potassium ions and water, controlling the evaporation-off operation on the basis of measurement data on the temperatures of the emission pipe and parts connected to the reactor vessel, and focused on the technology for synthesizing 18F-FDG and the technology for producing 18F fluoride ions.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2006241059 A
- JP HEI6157572 B
- JP HEI11508923 PCT
- JP HEI5345731 B
- JP 2005352464 A

### Non-patent literature cited in the description

- **Hamacher K. ; Coenen H. H. ; Stocklin G.** Efficient Stereospecific Synthesis of No-carrier-added-2-[18F]fluoro-2-deoxy-D-glucose Using Aminopolyether Supported Nucleophilic Substitution. *J. Nucl. Med.,* 1986, vol. 27 (2), 235-238
- Computer-aided Synthesis (CAS) of No-carrier-added 2-[18F]Fluoro-2-deoxy-D-glucose: an Efficient Automated System for the Aminopolyether-supported Nucleophilic Fluorination. **K. Hamacher et al.** Applied Radiation and Isotopes (Great Britain). Pergamon Press, 1990, vol. 41, 49-55
- *Radioisotopes,* 2001, vol. 50, 205-227
- *Radioisotopes,* 2001, vol. 50, 228-256
- PET - A guideline to synthesis and clinical use
- **Radioisotopes.** *Radioisotopes,* 2001, vol. 50, 205-227